# EUROPEAN PATENT SPECIFICATION

(11) **EP 2 215 270 B1**
(45) Date of publication and mention of the grant of the patent: **09.01.2013**
(21) Application number: 08842775.2
(22) Date of filing: 24.10.2008
(51) Int. Cl.: G01N 33/569

(54) **DIAGNOSTIC AND THERAPEUTIC PROTOCOLS**
DIAGNOSE- UND THERAPIEVORSCHRIFTEN
PROTOCOLES DIAGNOSTIQUES ET THÉRAPEUTIQUES

(30) Priority: 26.10.2007 US 983062 P
(43) Date of publication of application: 11.08.2010
(73) Proprietor: Alfred Health, Melbourne, Victoria 3004 (AU)
(72) Inventor: LEWIN, Sharon R, Armadale Victoria 3143 (AU); CORNALL, Alyssa Marie, Nightcliff Northern Territory 0810 (AU); CAMERON, Paul Urquhart, Northcote Victoria 3070 (AU)
(74) Representative: Jones, Elizabeth Louise
(86) International application number: PCT/AU2008/001578
(87) International publication number: WO 2009/052579

(56) References cited:
- US-B2- 6 727 060
- SHALEKOFF SHARON ET AL: "Duration of sample storage dramatically alters expression of the human immunodeficiency virus coreceptors CXCR4 and CCR5", CLINICAL AND DIAGNOSTIC LABORATORY IMMUNOLOGY, vol. 8, no. 2, March 2001 (2001-03), pages 432-436, XP002603430, ISSN: 1071-412X
- TROUPLIN V ET AL.: 'Determination of coreceptor usage of human immunodeficiency virus type 1 from patient plasma samples by using a recombinant phenotypic assay' JOURNAL OF VIROLOGY vol. 75, no. 1, 2001, pages 251 - 259, XP002183818
- BRAUN P ET AL.: 'Phenotypic assays for the determination of coreceptor tropism in HIV- infected individuals' EUROPEAN JOURNAL OF MEDICAL RESEARCH vol. 12, no. 9, 15 October 2007, pages 463 - 472, XP008127662
- TURGEON ML.: 'Clinical Hematology, Theory & Procedures.', 1988, LITTLE, BROWN AND COMPANY, BOSTON/TORONTO, ISSN 0-316-856 page 6 AND 7, XP008134380

### SEQUENCE LISTING

<110> ALFRED HEALTH
   MONASH UNIVERSITY
   Lewin, Sharon
   Cornall, Alyssa
   Cameron, Paul
<120> DIAGNOSTIC AND THERAPEUTIC PROTOCOLS
<130> 30669544/HPM
<150> US 60/983062
   <151> 2007-10-26
<160> 3
<170> PatentIn version 3.4
<210> 1
   <211> 23
   <212> DNA
   <213> primer
<400> 1
   tctctagcag tggcgcccga aca 23
<210> 2
   <211> 23
   <212> DNA
   <213> primer
<400> 2
   tctccttcta gcctccgcta gtc 23
<210> 3
   <211> 37
   <212> DNA
   <213> primer
<400> 3
   cgggagtact caccagtcgc cgcccctcgc cctcccg 37

## Description

### FIELD

The present invention relates generally to the fields of diagnostic assays and therapeutic protocols with respect to viral infection.

### BACKGROUND

Bibliographic details of references provided in the subject specification are listed at the end of the specification.

Reference to any prior art is not, and should not be taken as an acknowledgment or any form of suggestion that this prior art forms part of the common general knowledge in any country.

Infection by human immunodeficiency virus -1 (HIV-1 or HIV) can lead to serious morbidity and life threatening disease conditions, including acquired immunodeficiency disease syndrome (AIDS). Whilst some treatments such as highly active anti-retroviral therapy (HAART) have been successful in controlling HIV-1 infection, significant numbers of patients fail treatment. This is most often due to the development of drug resistance or an impaired immunological recovery. Individuals who fail HAART in the setting of drug resistance are often infected with a virus with enhanced replicative capacity (Solomon et al. Journal of Infectious Diseases 187:1915-1923, 2003; Solomon et al. Journal of Acquired Immune Deficiency Syndrome 40:140-148, 2005).

Shalekoff and Tiemessen Clinical and Diagnostic Laboratory Immunology 8(2):432-436, 2001 discusses how duration of sample storage of whole blood dramatically alters expression of the Human Immunodeficiency Virus Co-receptors CXCR4 and CCR5. US 6727060 discusses analysis of HIV-1 co-receptor use in the clinical care of HIV-1-infected - patients. Trouplin et al. Journal of Virology 75:251-259, 2001 discusses the determination of co receptor usage of human immunodeficiency virus type 1 from patient plasma samples by using a recombinant phenotype assay. Braun and Wiesmann European Journal of Medical Research 12:463-472, 2007 discusses phenotypic assays for the determination of co-receptor tropism in HIV-infected individuals.

In order to infect a CD4⁺ T-cell, HIV-1 first binds to surface molecule CD4 and then binds to a co-receptor. The most commonly used co-receptors are CCR5 and CXCR4. Viruses that use CCR5 are referred to as R5 viruses while those that use CXCR4 are X4 viruses. X4 viruses are more common in patients with AIDS and have been associated with disease progression, including patients who have failed HAART *(Solomon et al.* 2005 *supra*). Drugs that block entry of the virus into the cell by either inhibiting fusion or binding to the CCR5 molecule are now available (Cooley et al. J Clin Virol 26(2):121-132, 2003). Compounds that inhibit binding to CCR5 (CCR5 antagonist) are only active in individuals infected with R5 viruses. Therefore, rapid assessment of the co-receptor use of patient virus is critical before initiating therapy with this class of compounds.

There are a number of assays available to measure replicative ability and co-receptor use. However, none of these assays can reproducibly assess virus directly from patient blood and/or are time-consuming and labor intensive. Indeed in the one assay that proposes direct detection of HIV from patient plasma, detection of virus directly from plasma is often complicated by clot formation *in vitro.*

Consequently, phenotypic co-receptor and fitness assays require initial amplification of virus from patient peripheral blood mononuclear cells (PBMCs). This process is time consuming (on average taking 1-2 weeks), only has 70-80% sensitivity (meaning only 70% of viruses with greatest "fitness" will actually grow in donor PBMC) and will amplify the dominant circulating virus which is not necessarily representative of the mix of circulating quasispecies. Culturing virus in donor PBMC also selects for strains which replicate well in PBMCs. Sensitivity for detection of virus following amplification from patient plasma is even lower, at around 30-50%.

There is a need for a rapid assay to determine co-receptor usage of viruses and viral load in a reproducible manner over a range of viral loads.

Such an assay is useful in the diagnosis and therapy of viral infection and in particular infection by HIV.

### SUMMARY

As discussed below, the present invention provides a diagnostic assay to determine co-receptor usage of viruses and in particular HIV. Such an assay enables the development of therapeutic protocols based on targeting a particular combination of receptors used by viruses. Hence, the present invention facilitates diagnostic and therapeutic protocols based on co-receptor usage of viruses and in particular HIV.

The instant invention is predicated in part on the ability to assay HIV from a patient's plasma for co-receptor usage without the need for amplification of viruses in donor peripheral blood mononuclear cells (PBMC). The assay of the invention employs indicator cell lines which express CD4 and one or both of CCR5 and CXCR4.

Accordingly, the present invention provides a method for determining co-receptor usage of HIV in a subject, the method comprising culturing, in the presence of an anti-coagulant, a viral preparation derived from the subject's plasma with one or more cell lines selected from:
(i) cells which express surface CD4 and CCR5;
(ii) cells which express surface CD4 and CXCR4; and
(iii) cells which express surface CD4, CCR5 and CXCR4; wherein the anti-coagulant is added to the viral preparation or to the culture;
and screening for infection, wherein the presence or absence of infected cells is indicative of the co-receptors used by the HIV.

In an embodiment, the viral preparation is derived from a subject's plasma collected in the presence of an anti-coagulant. This may be the same or different to the added anti-coagulant.

Hence, another aspect of the present invention provides a method for determining co-receptor usage of an HIV in a subject, the method comprising culturing a viral preparation derived from the subject's plasma, the plasma being collected in the presence of an anti-coagulant which may be the same or different from the first mentioned (added) anti-coagulant, with cell lines selected from
(i) cells which express surface CD4 and CCR5;
(ii) cells which express surface CD4 and CXCR4; and
(iii) cells which express surface CD4, CCR5 and CXCR4; wherein the added anti-coagulant is added to the viral preparation or to the culture.
and screening for infection wherein the presence or absence of infected cells is indicative of the co-receptors used by the HIV.

In a further aspect the present invention is also directed to a method for determining co-receptor usage of an HIV in a subject, the method comprising collecting blood from the subject in the presence of an anti-coagulant, generating plasma from the blood, culturing virus isolated from the plasma with cell lines selected from:
(i) cells which express surface CD4 and CCR5;
(ii) cells which express surface CD4 and CXCR4; and
(iii) cells which express surface CD4, CCR5 and CXCR4; wherein an anti-coagulant which may be the same as or different to the first anti-coagulant is added to the viral preparation or to the culture
and screening for infection wherein the presence or absence of infected cells is indicative of the co-receptors used by the HIV.

Reference to "HIV" includes HIV-1 and related viruses. Furthermore, reference to "CD4", "CCR5" and CXCR4" includes homologs, derivatives and functional equivalents thereof.

The term "anti-coagulant" is used in its broadest sense to encompass anti-coagulants and agents having an anti-coagulant effect such as anti-platelet drugs, thrombolytic and fibrinolytic drugs, and non-medicinal agents such as citrate, citrate dextrose (ACD), EDTA and oxalate. In addition, other techniques can be used which prevent clotting. These include separation of pro-coagulant factors and viral factors, removal of coagulant pathway factors, and preventing interaction between coagulation factors and their ligands. Conveniently, the anti-coagulant is selected from the list comprising heparin, ACD and EDTA or maybe a combination of two or more anti-coagulant agents such as heparin and ACD, heparin and EDTA, ACD and EDTA and heparin, ACD and EDTA. The first and second mentioned anti-coagulant steps may use the same or different anti-coagulants or combinations of anti-coagulants. The subject's blood includes whole blood which is generally collected in the presence of an anti-coagulant and further anti-coagulant added during the culturing phase.

The assay of the present invention does not require prior viral amplification. Infection of indicator cells can be by a centrifugation referred to herein as "spinoculation". This aids in maximizing infections of the indicator cell lines. Optionally, a positively charged polymer is also added such as polybrene (1,5-dimethyl-1,5-diazaundecamethylene polymethobromide, hexadimethrine bromide). Alternatively, SEVI can be added to the culture step. In methods of the invention, the use of spinoculation, an anti-coagulant and optionally a cationic polymer can minimize clot formation and maximize viral infection and can be used in the manufacture of an assay to determine co-receptor usage by HIV.

The assay of the present invention further enables the determination of viral loads for any virus. Accordingly, the present invention provides a method for determining viral infection (load) in a subject, the method comprising culturing virus isolated from plasma from the subject with cells capable of being infected by the virus, adding an anti-coagulant to the virus/cell culture, screening for infection and then determining viral genome copy number in infected cells or culture supernatant.

Therapeutic protocols are also contemplated herein by first determining co-receptor usage by a virus and then selecting an appropriate agent which targets one or more of the co-receptors. Similarly, the assay herein is useful for detecting any change in viral strain during therapy leading to an alternation in co-receptor usage and therefore a change in therapeutic protocol.

Kits for conducting methods of the invention also form part of the present invention.

### BRIEF DESCRIPTION OF THE FIGURES

Some figures contain color representations or entities. Color photographs are available from the Patentee upon request or from an appropriate Patent Office. A fee may be imposed if obtained from a Patent Office.

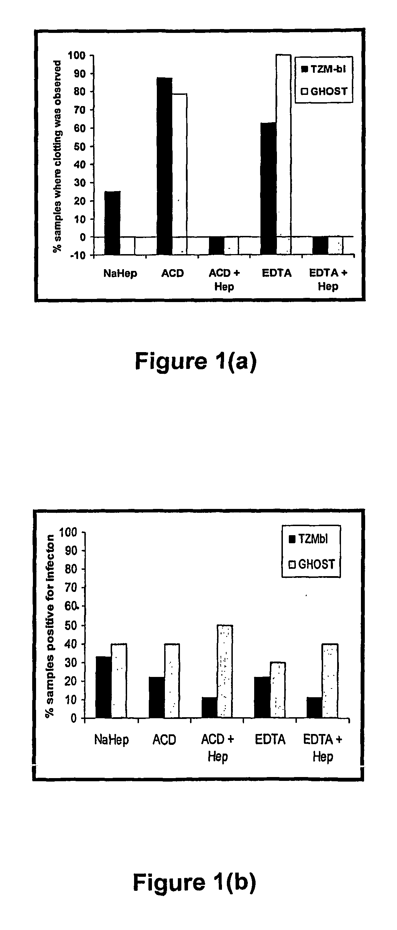

**Figures 1(a) and (b)** are graphical representations showing (a) clot formation and (b) infection of indicator cell lines. **(a)** Percentage of patient plasma samples that clot following collection with different anti-coagulants and infection of TZM-bl cells (black; n = 8) and GHOST cells (grey; n = 14). Infection in the TZM-bl and GHOST cell line was determined by detection of β-gal expression and GFP, respectively. **(b)** Percentage of patient plasma that infect either TZM-bl cells (black; n = 9) or GHOST cells (grey; n = 10). All samples were matched i.e. the same plasma from each patient was collected in each anti-coagulant which included: NaHep - blood was collected in sodium heparin; ACD - blood was collected in ACD; ACD + Hep - blood was collected in ACD and then heparin added to the pelleted plasma; EDTA - blood was collected in EDTA; EDTA + Hep - blood was collected in EDTA and then Heparin was added to the pelleted plasma. The data presented in this figure are generated from the protocol as stated, however, storage time and temperature of storage varied between 2 and 24 hours. In addition, some patient samples were frozen at -80°C while others were processed immediately. Infection was detected using microscopy as appropriate per indicator cell line used.

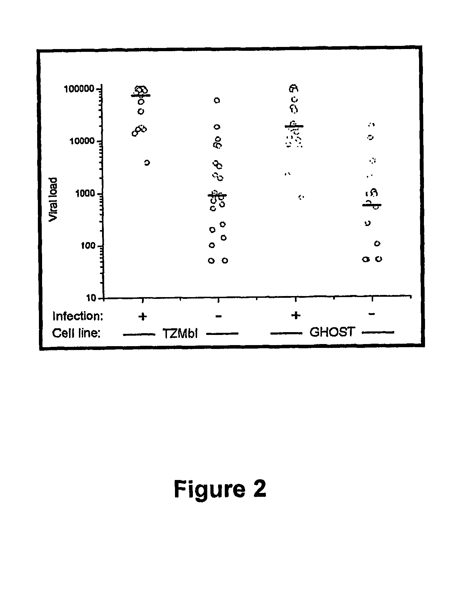

**Figure 2** is a diagrammatic representation showing relationship of plasma vial load (y axis, copies/ml) to the detection of infection by microscopy using either TZMB1 (blue) or GHOST cells (green) as indicator cell lines (n=33). The horizontal line demonstrates the median viral load. The data presented in this figure are generated from the protocol as stated, however, storage time of plasma prior to preparation varied between 2 and 24 hours. In addition, some patient samples were frozen at -80 °C while others were processed immediately. Infection was detected using microscopy as appropriate per indicator cell line used.

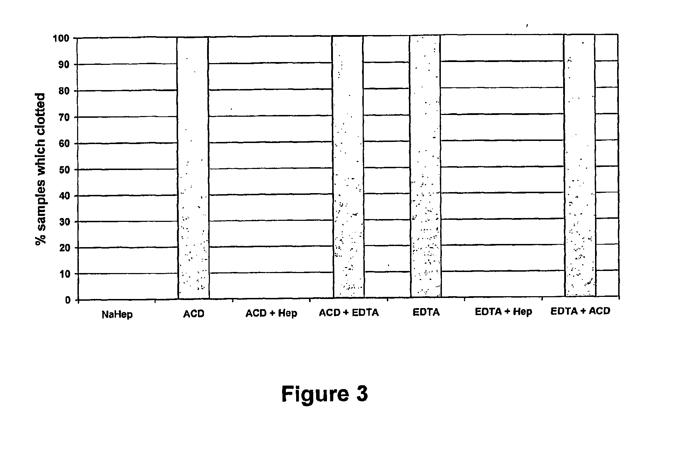

**Figure 3** is a graphical representation showing effect of anti-coagulant on clotting of patient plasma samples during infection (n=5). The data presented in this figure are generated from the protocol as stated, however, storage time and temperature of storage varied between 2 and 24 hours. In addition, some patient samples were frozen at -80°C while others were processed immediately. Infection was detected using microscopy as appropriate per indicator cell line used.

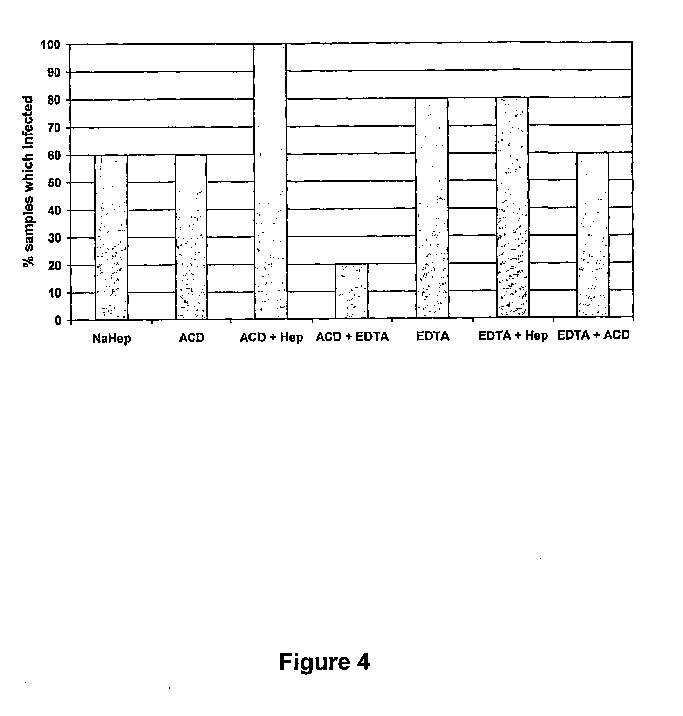

**Figure 4** is a graphical representation showing effect of anti-coagulant on ability of patient plasma to infect GHOSTX4R5 cells (n=5). The data presented in this figure are generated from the protocol as stated, however, storage time and temperature of storage varied between 2 and 24 hours. In addition, some patient samples were frozen at -80°C while others were processed immediately. Infection was detected using microscopy as appropriate per indicator cell line used.

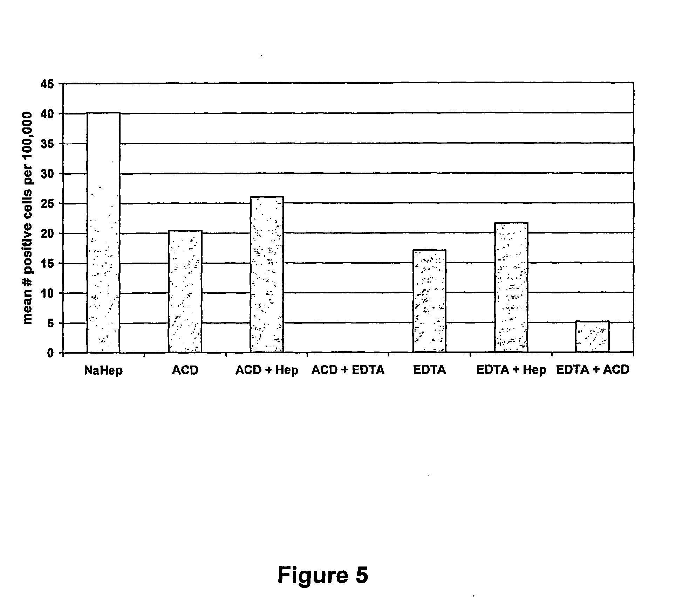

**Figure 5** is a graphical representation showing effect of anti-coagulant on efficiency of infection of GHOSTX4R5 cells with HIV-1 patient plasma (n=5). The data presented in this figure are generated from the protocol as stated, however, storage time and temperature of storage varied between 2 and 24 hours. In addition, some patient samples were frozen at -80°C while others were processed immediately. Infection was detected using microscopy as appropriate per indicator cell line used.

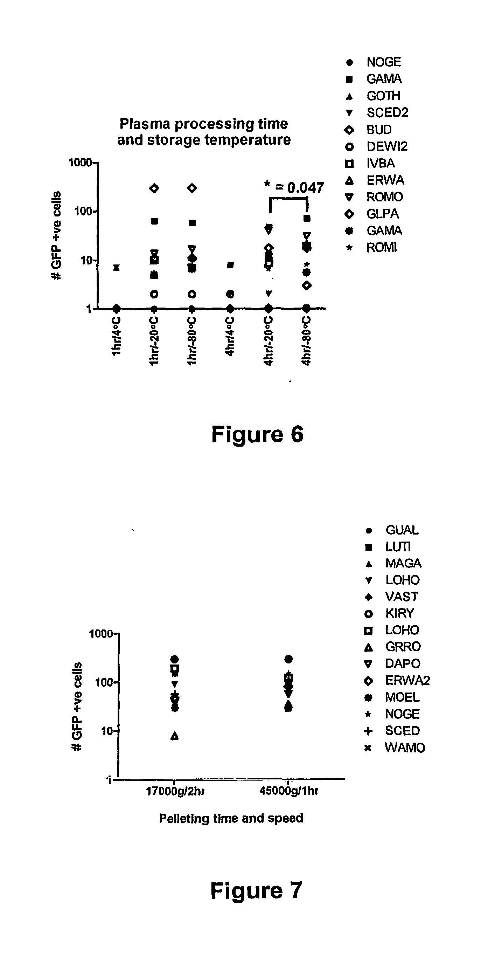

**Figure 6** is a diagrammatic representation of the number of GFP positive cells detected when plasma samples were prepared within either 1 or 4 hours of collection and stored at either 4°C, -20°C or -80°C. Each symbol represents a different patient.
**Figure 7** is diagrammatic representation of the number of GFP positive cells detected when plasma was pelleted either at 17000g for 2hr or 45000g for 1hr. Each symbol represents a different patient.

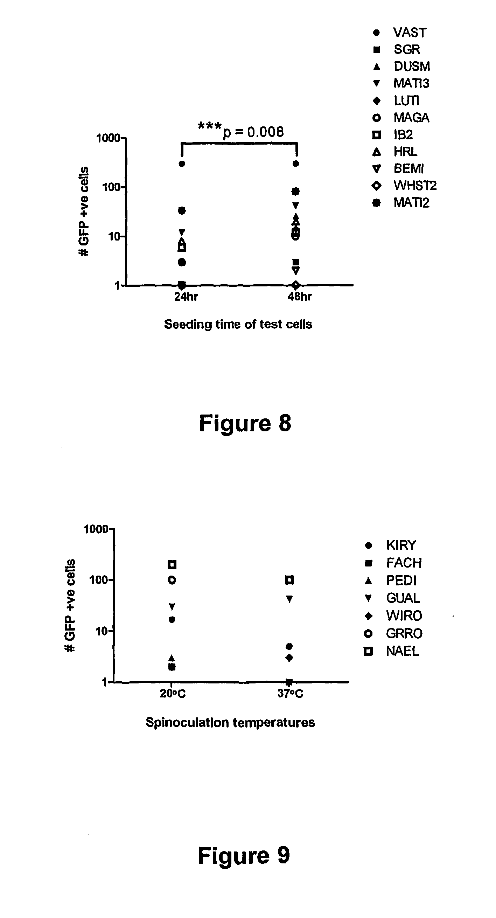

**Figure 8** is diagrammatic representation of the number of GFP positive cells detected when GHOST X4R5 cells were seeded either 24hrs or 48 hrs prior to infection. Each symbol represents a different patient.
**Figure 9** is diagrammatic representation of the number of GFP positive cells detected when GHOST X4R5 cells were spinoculated at either 20°C or 37°C. Each symbol represents a different patient.

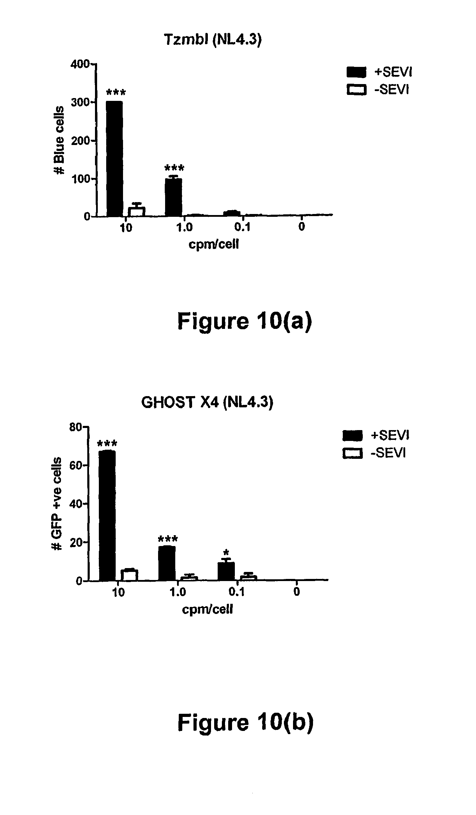

**Figures 10(a) and (b)** are graphical representations of the number of blue cells or GFP positive cells detected when Tzmbls or GHOST X4 cells were infected with the laboratory strain NL4.3, which utilises X4 (10.0, 1.0, 0.1, 0.0 cpm/cell) in the presence or absence of SEVI fibrils (50µg/ml). The values are presented as the mean ± SEM. *** means p < 0.001 and * means p < 0.05 by two-way annova.

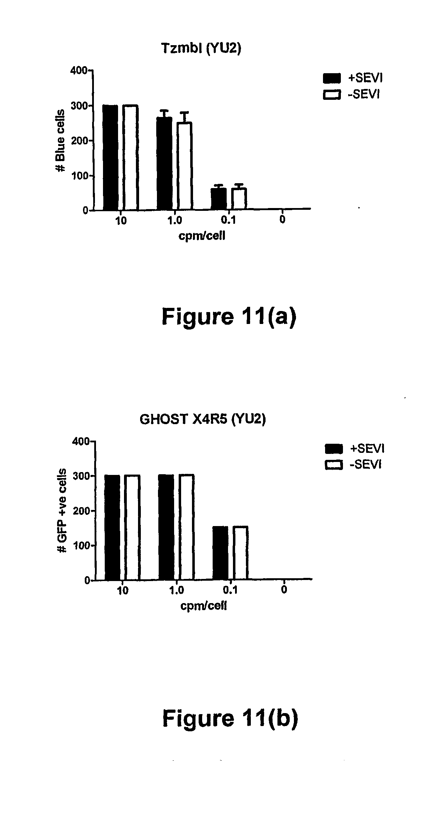

**Figure 11 (a) and (b)** are graphical representations of the number of blue cells or GFP positive cells detected when Tzmbls or GHOST X4/R5 cells were infected with the laboratory YU2 virus which utilises R5 (10.0, 1.0, 0.1, 0.0 cpm/cell) in the presence or absence of SEVI fibrils (50µg/ml). The values are presented as the mean ± SEM.

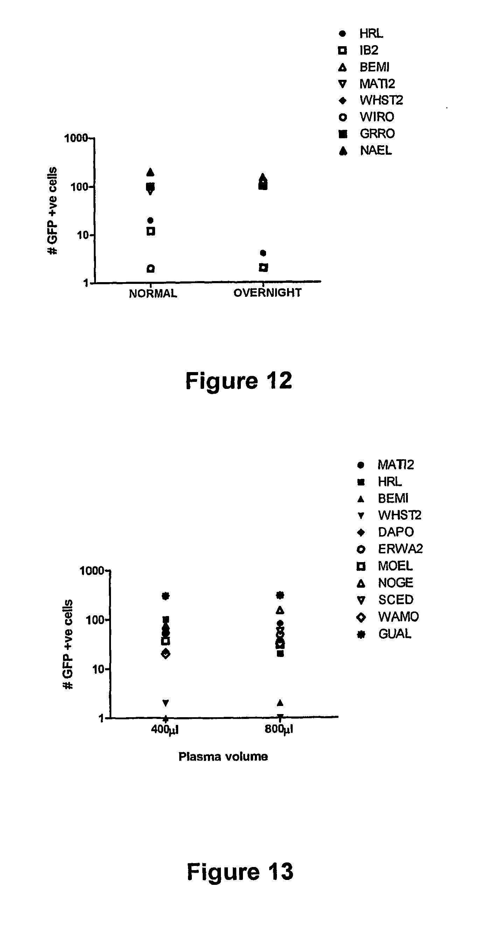

**Figure 12** is a diagrammatic representation of the number of GFP positive cells detected when media is added either immediately after spinoculation or after overnight incubation at 37°C. Each symbol represents different patient.
**Figure 13** is a diagrammatic representation of the number of GFP positive cells detected when GHOST X4R5 cells were infected with starting plasma volume either 400µl or 800µl. Each symbol represents a different patient.

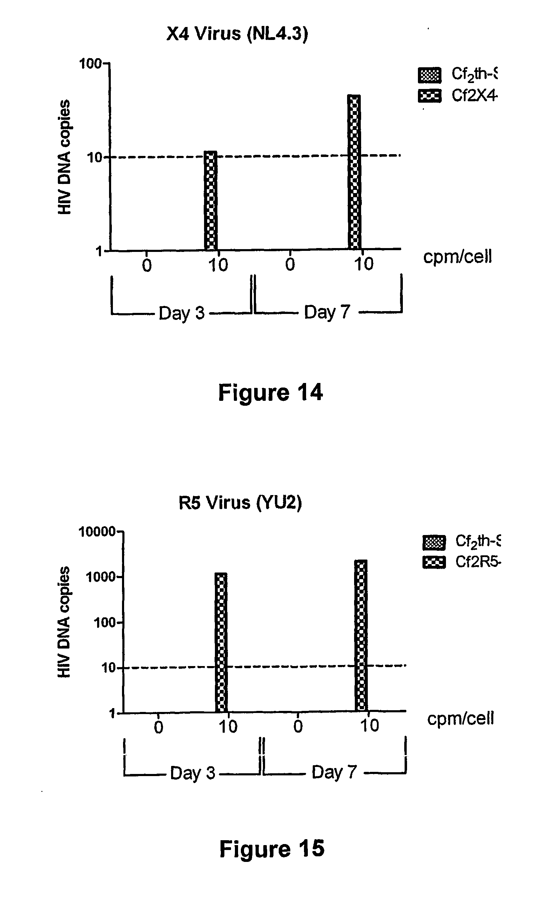

**Figure 14** is a graphical representation showing the time dependent increase in HIV DNA copies when Cf₂th/CD4X4 (Cf₂X4) were infected with X4 using NL4.3 virus. Cf₂th (no CD4 or CXCR4) and Cf₂X4 (CD4 and CXCR4) cells were seeded 24 hrs prior to infection. After 24 hrs cells were infected with an X4 virus (NL4.3) at 10 or 0 cpm/cell. At Day 3 and Day 7 cells were washed three times with PBS and the DNA lysates were prepared. The lower detection limit of the assay is represented by the dotted line which is 10 copies.
**Figure 15** is a graphical representation showing the time dependent increase in HIV DNA copies when Cf₂th/CD4R5 (Cf₂R5) were infected with R5 using YU2 virus. Cf₂th (no CD4 or CCR5) and Cf₂R5 (CD4 and CCR5) cells were seeded 24 hrs prior to infection. After 24 hrs cells were infected with an R5 virus (YU2) at 10 or 0 cpm/cell. At Day 3 and Day 7 cells were washed three times with PBS and the DNA lysates were prepared. The lower detection limit of the assay is represented by the dotted line which is 10 copies.

### DETAILED DESCRIPTION

The singular forms "a", "an" and "the" include plural aspects unless the context clearly dictates otherwise. Thus, for example, reference to "a virus" includes a single virus as well as two or more viruses; reference to "an anti-coagulant" includes a single anti-coagulant, as well as two or more anti-coagulants; reference to "the invention" includes a single invention or multiple aspects of an invention; and so forth.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

As described above, the present invention provides in one embodiment a method for determining HIV co-receptor usage. The determination of co-receptor usage enables selection of appropriate therapeutic agents such as CCR5 antagonists or CXCR4 antagonists for the treatment of HIV infection. Hence, patients are subject to effective anti-viral therapy. Reference to "HIV" in this context includes HIV-1 and related viruses such as HIV-2 and HTLV. Generally, the subject is a human. However, non-human animal models are encompassed by the present invention and hence the subject may also be a non-human primate or mammal. In this context, therefore, the present invention extends to simian, canine and feline immunodeficiency viruses as well as non-human animal models.

The present invention is predicated in part on the use of indicator cell lines expressing surface CD4 and one or both of CCR5 and CXCR4 in an assay of plasma potentially comprising HIV. Infection of the indicator cell lines indicates co-receptor usage in the form of CD4/CCR5 or CD4/CXCR4 or CD4/CCR5/CXCR4. Reference to "CD4", "CCR5" and "CXCR4" includes homologs, derivatives and functional equivalents thereof.

Accordingly, the present invention comprises a method for determining co-receptor usage of an HIV in a subject, the method comprising culturing a viral preparation derived from the subject's plasma with one or more cell lines selected from:
(i) cells which express surface CD4 and CCR5;
(ii) cells which express surface CD4 and CXCR4; and
(iii) cells which express surface CD4, CCR5 and CXCR4; wherein an anti-coagulant is added to the viral preparation or to the culture;
and screening for infection wherein the presence or absence of infected cells is indicative of the co-receptors used by the HIV.

In an embodiment, the viral preparation is derived from a subject's plasma collected in the presence of an anti-coagulant. This may be the same or different to the added anti-coagulant.

Hence, another aspect of the present invention provides a method for determining co-receptor usage of an HIV in a subject, the method comprising culturing a viral preparation derived from the subject's plasma, the plasma being collected in the presence of an anti-coagulant which may be the same or different from the added anti-coagulant, with cell lines selected from:
(i) cells which express surface CD4 and CCR5;
(ii) cells which express surface CD4 and CXCR4; and
(iii) cells which express surface CD4, CCR5 and CXCR4; wherein the added anti-coagulant is added to the viral preparation or to the culture;
and screening for infection wherein the presence or absence of infected cells is indicative of the co-receptors used by the HIV.

The viral preparation is from the subject's plasma which is prepared in the presence of an anti-coagulant.

The present invention is also directed to a method for determining co-receptor usage of an HIV in a subject, the method comprising collecting blood from the subject in the presence of an anti-coagulant, generating plasma from the blood, isolating a viral preparation from the plasma, culturing the viral preparation with cell lines selected from:
(i) cells which express surface CD4 and CCR5;
(ii) cells which express surface CD4 and CXCR4; and
(iii) cells which express surface CD4, CCR5 and CXCR4; wherein an anti-coagulant, which is the same or different to the first anti-coagulant, is added to the viral preparation or the culture;
   and screening for infection wherein the presence or absence of infected cells is indicative of the co-receptors used by the HIV.

Also disclosed is a method for determining co-receptor usage of an HIV in a subject, the method comprising culturing a viral preparation isolated from plasma from the subject in the presence of an anti-coagulant with cells which express surface CD4 and one or both of CCR5 and CXCR4, the plasma comprising an anti-coagulant and then screening cells for infection wherein the presence of infection is indicative of particular co-receptors.

Accordingly, one aspect of the present invention comprises a method for determining co-receptor usage of an HIV in a subject, the method comprising culturing a viral preparation derived from the subject's plasma with one or more cell lines selected from:
(i) cells which express surface CD4 and CCR5;
(ii) cells which express surface CD4 and CXCR4; and
(iii) cells which express surface CD4, CCR5 and CXCR4; wherein an anti-coagulant is added to the viral preparation or to the culture;
and screening for infection wherein the presence or absence of infected cells is indicative of the co-receptors used by the HIV.

In an embodiment, the viral preparation is derived from a subject's plasma collected in the presence of an anti-coagulant. This may be the same or different to the added anti-coagulant.

Hence, another aspect of the present invention provides a method for determining co-receptor usage of an HIV in a subject, the method comprising culturing a viral preparation derived from the subject's plasma, the plasma being collected in the presence of an anti-coagulant which may be the same or different from the added anti-coagulant, with cell lines selected from:
(i) cells which express surface CD4 and CCR5;
(ii) cells which express surface CD4 and CXCR4; and
(iii) cells which express surface CD4, CCR5 and CXCR4; wherein the added anti-coagulant is added to the viral preparation or to the culture;
and screening for infection wherein the presence or absence of infected cells is indicative of the co-receptors used by the HIV.

Reference to an "anti-coagulant" includes classes of anti-coagulants such as anti-coagulants, anti-platelet drugs, thrombolytic and fibrinolytic drugs and metal ion chelators agents such as citrate, citrate dextrose (ACD) and EDTA and oxalate.

In a related aspect, the anti-coagulants include heparin and glycosaminoglycans such as low molecular weight heparin such as Bemiparin, Certoparin, Dalteparin, Enoxaparin, Nadroparin, Parnaparin, Reviparin and Tinzaparin and heparinoid such as Danaparoid, Sulodexide, Dermatan sulfate; direct thrombin (II) inhibitors such as Argatroban, Bivalirudin, Dabigatran, Desirudin, Hirudin, Lepirudin, Melagatran, Ximelagatran; Fact Xa inhibitors (such as Tick Anticoagulant Peptide), such as Apixaban, Otamixaban, Rivaroxaban and oligosaccharides such as Fondaparinux and Idraparinux; Vitamin K antagonists such as Acenocoumarol, Clorindione, Coumatetralyl, Dicoumarol (Dicumarol), Diphenadione, Ethyl biscoumacetate, Phenprocoumon, Phenindione, Tioclomarol and Warfarin.

Examples of anti-platelet drugs include Glycoprotein IIb/IIIa inhibitors such as Abciximab, Eptifibatide, Tirofiban; ADP receptor/P2Y12 inhibitors such as Clopidogrel, Ticlopidine, and Prasugrel and other Thienopyridines; Prostaglandin analogs such as Beraprost, Prostacyclin, Iloprost, Treprostinil; Cox inhibitors such as Acetylsalicylic acid, Aspirin, Aloxiprin, Carbasalate calcium; as well as Ditazole, Cloricromen, Dipyridamole, Indobufen, Picotamide, Triflusal.

In addition, coagulation can be prevented using the following methods and agents:
(1) **Separation** of pro-coagulant factors and viral particles by Ligand Affinity:
   (a) Whereby the ligand is a ligand of the coagulation factor (I, II, V, VII, VIII, IX, X, XI and XII.); or
   (b) Whereby the ligand is ligand of the viral particle (e.g. CD4)
   wherein the ligand is immobilized or the ligand and its counter part are separatable, for example by size exclusion or density or charge or tag or affinity to antibody (e.g. FC region) i.e. immunoprecipitation or affinity chromatography.
(2) **Removing** key coagulant pathway factors (blood coagulation factor I, II, V, VII, VIII, IX, X, XI and XII) from the plasma prior to generation of the plasma fraction (viral isolate preparation) by providing antibodies to coagulant factors (blood coagulation factor antibodies: I, II, V, VII, VIII, IX, X, XI and XII.) said antibodies;
   (a) being bound to a column and plasma passed over the column
   (b) bound to a bead and added directly to plasma and subsequently retrieved
   (c) added directly to plasma and retrieving antibody-target complex by any means
   e.g. separated by size exclusion or Fc affinity chromatography or by other mentioned below and known to persons skilled in the art.
(3) **Preventing** interaction of coagulation factors and their ligand by adding antibodies to coagulation enzymes of their coagulation enzyme ligand.
**(4) Providing inhibitors** of the coagulation factors, whereby the inhibitors are:
   (a) Generated by the addition of another factor (as per US Patent No. 6,627,193)
   (b) Added directly, such inhibitors being 'natural' inhibitors of from orthologous or homologous species, where such inhibitors can be:
      *i. chemical entities*
         o FXa inhibitors, e.g. sulfonamide lactam inhibitors
         o FXa inhibitors, e.g. Sulfonamidopyrrolidinone
         o Melagatran (and its prodrug ximelagatran); Ximelagatran (Exanta or Exarta, H 376/95) direct thrombin inhibitor
         o Argatroban
         o Dabigatran
      *ii. polypeptide*
         (i) serine protease inhibitors
            1. Heparin Cofactor II
            2. Tissue Factor pathway inhibitor
            3. kunitz type protease inhibitors(eg tick anticoagulant protein)
            4. Antithrombin III Glycoprotein
         (ii) Protein C is a major physiological anticoagulant. It is a vitamin K-dependent serine protease enzyme (EC 3.4.21.69) that is activated by thrombin into activated protein C (APC). The activated form (with protein S and phospholipid as a cofactor) degrades Factor Va and Factor VIIIa.
         (iii) Diect Thrombin Inhibitors
            1. hirudins and the like
            2. Bivalirudin (transient inhibition - is cleaved by thrombin)
            3. Lepirudin
            4. Desirudin
      *iii polysaccharides*
         (a) Fondaparinux is a synthetic sugar composed of the five sugars (pentasaccharide) in heparin that bind to antithrombin. It is a smaller molecule than low molecular weight heparin.
         (b) Idraparinux
         (c) Heparin Heparin, a highly-sulfated glycosaminoglycan Native heparin is a polymer with a molecular weight ranging from 3 kDa to 50 kDa, although the average molecular weight of most commercial heparin preparations is in the range of 12 kDa to 15 kDa. Heparin is a member of the glycosaminoglycan family of carbohydrates (which includes the closely-related molecule heparan sulfate) and consists of a variably-sulfated repeating disaccharide unit.
         (d) Deramatan Sulfe
(5) **Metal Ion Chelators** e.g. EDTA, ACD, Oxalate
   Chemical entities that work by binding calcium ions, preventing the coagulation proteins from using them:
   EDTA
   Citrate. It can be in the form of sodium citrate or ACD.
   Oxalate has a mechanism similar to that of citrate.

As used herein, use of the term anti-coagulant includes not only the agents/compounds listed herein, but also encompasses methods that prevent coagulation.

In one aspect, the anti-coagulant added to the indicator cell line/viral culture is not a metal ion chelator. Metal ion chelators include, without being limited to, ACD, EDTA, oxalate, or any other compounds with a similar mode of action.

Examples of thrombolytic and fibrinolytic agents include *plasminogen activators* such as TPA (Alteplase, Reteplase, Tenecteplase), UPA (Urokinase, Saruplase), Streptokinase, Anistreplase, Monteplase; and *serine endopeptidases,* such as Ancrod, Fibrinolysin; as well as Brinase.

Particular anti-coagulants include heparin, ACD or EDTA or a combination of two or more anti-coagulants such heparin/ACD, heparin/EDTA, ACD/EDTA or heparin/ACD/EDTA. The same or different anti-coagulants may be used at the blood collection stage and the indicator cell/viral preparation culturing stage.

In a related aspect, the anti-coagulants contemplated by the methods of the present invention exclude metal ion chelators, such as ACD, EDTA and oxalate or derivatives thereof.

Another aspect of the present invention comprises a method for determining co-receptor usage of an HIV in a subject, the method comprising culturing a viral preparation derived from the subject's plasma with one or more cell lines selected from:
(i) cells which express surface CD4 and CCR5;
(ii) cells which express surface CD4 and CXCR4; and
(iii) cells which express surface CD4, CCR5 and CXCR4; wherein an anti-coagulant selected from the list comprising heparin; ACD; EDTA; heparin and ACD; heparin and EDTA; and heparin, ACD and EDTA is added to the viral preparation or the culture;
and screening for infection wherein the presence or absence of infected cells is indicative of the co-receptors used by the HIV.

The present invention also provides a method for determining co-receptor usage of an HIV in a subject, the method comprising culturing a viral preparation derived from the subject's plasma, the plasma being collected in the presence of an anti-coagulant selected from the list comprising heparin; ACD; EDTA; heparin and ACD; heparin and EDTA; and heparin, ACD and EDTA, wherein the anti-coagulant may be the same or different from the first mentioned (added) anti-coagulant, with cell lines selected from:
(i) cells which express surface CD4 and CCR5;
(ii) cells which express surface CD4 and CXCR4; and
(iii) cells which express surface CD4, CCR5 and CXCR4; wherein the added anti-coagulant is selected from the list comprising heparin; ACD; EDTA; heparin and ACD; heparin and EDTA; and heparin, ACD and EDTA is added to the viral preparation or the culture;
and screening for infection wherein the presence or absence of infected cells is indicative of the co-receptors used by the HIV.

Preferred embodiments of the invention are provided below in which the named anti-coagulant is added to the vial preparation or to the culture.

Thus, another aspect of the present invention provides a method for determining co-receptor usage of an HIV in a subject, the method comprising culturing, in the presence of heparin plasma or viral preparation derived from the plasma of the subject with cells which express surface CD4 and one or both of CCR5 and CXCR4, and then screening cells for infection is indicative of particular co-receptors.

The present invention also provides a method for determining co-receptor usage of an HIV in a subject, the method comprising culturing, in the presence of ACD, plasma or a viral preparation derived from the plasma of the subject with cells which express surface CD4 and one or both of CCR5 and CXCR4, and then screening cells for infection wherein the presence of infection is indicative of particular co-receptors.

Yet another aspect of the present invention provides a method for determining co-receptor usage of an HIV in a subject, the method comprising culturing, in the presence of EDTA, plasma or a viral preparation derived from the plasma of the subject with cells which express surface CD4 and one or both of CCR5 and CXCR4, and then screening cells for infection wherein the presence of infection is indicative of particular co-receptors.

Still another aspect of the invention provides a method for determining co-receptor usage of an HIV in a subject, the method comprising culturing, in the presence of heparin and ACD, plasma or a viral preparation derived from the plasma of the subject with cells which express surface CD4 and one or both of CCR5 and CXCR4, and then screening cells for infection wherein the presence of infection is indicative of particular co-receptors.

Even yet another aspect of the present invention provides a method for determining co-receptor usage of an HIV in a subject, the method comprising culturing, in the presence of heparin ad EDTA, plasma or a viral preparation derived from the plasma of the subject with cells which express surface CD4 and one or both of CCR5 and CXCR4, and then screening cells for infection wherein the presence of infection is indicative of particular co-receptors.

Still yet another aspect of the present invention provides a method for determining co-receptor usage of an HIV in a subject, the method comprising culturing, in the presence of heparin, EDTA and ACD, plasma or a viral preparation derived from the plasma of the subject with cells which express surface CD4 and one or both of CCR5 and CXCR4 and then screening cells for infection wherein the presence of infection is indicative of particular co-receptors.

In a related aspect, the present invention provides a method for determining co-receptor usage of an HIV in a subject, the method comprising culturing in the presence of an anti-coagulant plasma or a viral preparation derived from the plasma of the subject with cells which express surface CD4 and one or both of CCR5 and CXCR4, and then screening cells for infection wherein the presence of infection is indicative of particular co-receptors, wherein the anti-coagulant is not a metal ion chelator. Examples of metal ion chelators include EDTA, ACD and oxalate.

In one aspect of the invention, the subject's blood includes whole blood and is conveniently collected in a blood tube comprising the anti-coagulants such as heparin, ACD and/or EDTA, the plasma isolated and the virus concentrated by centrifugation of the plasma over a 20% w/v sucrose cushion. The viral preparation which is the resultant pellet from centrifugation is reconstituted and then added to the indicator cell line. Alternatively, the plasma is added directly to the indicator cell lines. Infection of the indicator cell lines (CD4/CCR5/CXCR4 and CD4/CCR5 or CD4/CXCR4) means that the virus utilises the receptors CD4/CCR5/CXCR4 or CD4/CCR5 or CD4/CXCR4.

In one aspect, the blood is collected from a subject in a tube containing an anti-coagulant. In a related aspect, the blood is then processed for plasma within 24 hours of collection, such as, for example, immediately after blood being collected or within 1, 2, 3, 4, 5, 6, 7, 8, 9, 10, 11, 12, 13, 14, 15, 16, 17, 18, 19, 20, 21, 22, 23 and 24 hours of the blood being collected.

At this stage, the plasma may be immediately processed for viral isolation. Alternatively, the plasma may be stored prior to viral isolation. Plasma storage may be at, for example, room temperature, 4°C, -20°C, or -80°C.

Viral isolation can be performed using any means known to the skilled artisan. In one embodiment, the plasma is layered over sucrose (for example 20% sucrose) and centrifuged. Centrifugation can be performed for any length of time and at any temperature suitable for pelleting the virus. For example, the centrifugation may be for 2 hours at 17,000 xg or 1 hour at 45,000 xg.

In one aspect, the indicator cell lines are adherent. The indicator cells can be seeded at anytime prior to the addition of the viral prepration. For example, cells may be seeded up to 24 hours or up to 48 hours prior to the addition of virus.

Spinoculation can also be used to aid in the infection process. For example, the virus and the indicator cell lines may be spun at 1200 xg at 37°C for 2 hours. Alternatively, the virus and indicator cell line may be spun at 20°C or colder.

In addition to the anti-coagulant being added to the viral preparation and indicator cell cultures, other factors may be added to enhance viral infectivity. For example, semen enhanced viral infectivity (SEVI) peptides and/or polybrene can also be added. Alternatively, the indicator cell line may be modified to express C-type lectin receptors, such as DC-sign and CD209.

Infection times of the indicator cell can be any time which allows the virus to infect the indicator cell line. For example, the virus may be left up to 24 hours prior to the addition of media. Alternatively, media can be added immediately following the addition of the virus or after the spinoculation.

The amount of patient plasma required to isolate the virus will vary, depending on, for example, the subject's viral load. For example, the methods of the present invention can be performed using 100µl, 200µl, 300µl, 400µl, 500µl, 600µl, 700µl, 800µl, 900µl, 1000µl, 1100µl, 1200µl, 1300µl, 1400µl, 1500µl, 1600µl, 1700µl, 1800µl, 1900µl and 2000µl of plasma.

Infection of indicator cell lines is enhanced by centrifugation (spinoculation). Generally, spinoculation comprises centrifugation at between 900 - 2000 xg for 30 minutes to 4 hours such as 1200 x g for 2 hours.

The present invention is further directed to a method for determining co-receptor usage of an HIV in a subject, the method comprising collecting blood from the subject in the presence of an anti-coagulant selected from the list comprising heparin; ACD; EDTA; heparin and ACD; heparin and EDTA; and heparin, ACD and EDTA, generating plasma from the blood, culturing a viral preparation derived from the subject's plasma with cell lines selected from:
(i) cells which express surface CD4 and CCR5;
(ii) cells which express surface CD4 and CXCR4; and
(iii) cells which express surface CD4, CCR5 and CXCR4; wherein an anti-coagulant, which may be the same as or different to the first anti-coagulant, selected from the list comprising heparin; ACD; EDTA; heparin and ACD; heparin and EDTA; and heparin, ACD and EDTA is added to the viral preparation or the culture;
and screening for infection wherein the presence or absence of infected cells is indicative of the co-receptors used by the HIV.

Additional preferred embodiments of the invention are provided below in which the anti-coagulant present during the culture step is added to the viral preparation or to the culture.

Thus, another aspect of the present invention provides a method for determining co-receptor usage of an HIV in a subject, the method comprising collecting blood for the subject in the presence of an anti-coagulant, generating plasma from the blood, culturing the plasma or the viral isolate derived from the plasma with cells which express surface CD4 and one or both of CCR5 and CXCR4, in the presence of an anti-coagulant and then screening cells for infection wherein the presence of infection is indicative of particular HIV co-receptors usage.

A further aspect of the present invention provides a method for determining co-receptor usage of an HIV in a subject, the method comprising collecting blood from the subject in the presence of an anti-coagulant, generating plasma from the blood, culturing the plasma or the viral isolate from the plasma with cells which express surface CD4 and one or both of CCR5 and CXCR4 in the presence of heparin and the screening cells for infection wherein the presence of infection is indicative of particular HIV co-receptor usage.

Yet another aspect of the present invention provides a method for determining co-receptor usage of an HIV in a subject, the method comprising collecting blood for the subject in the presence of an anti-coagulant, generating plasma from the blood, culturing the plasma or the viral isolate from the plasma with cells which express surface CD4 and one or both of CCR5 and CXCR4 in the presence of ACD and the screening cells for infection wherein the presence of infection is indicative of particular HIV co-receptor usage.

Still another aspect of the present invention provides a method for determining co-receptor usage of an HIV in a subject, the method comprising collecting blood from the subject in the presence of an anti-coagulant, generating plasma from the blood, culturing the plasma or the viral isolate derived from the plasma with cells which express surface CD4 and one or both of CCR5 and CXCR4 in the presence of EDTA and the screening cells for infection, wherein the presence of infection is indicative of particular HIV co-receptor usage.

Even yet another aspect of the present invention provides a method for determining co-receptor usage of an HIV in a subject, the method comprising collecting blood for the subject in the presence of an anti-coagulant, generating plasma from the blood, culturing the plasma or the viral isolate derived from the plasma with cells which express surface CD4 and one or both of CCR5 and CXCR4, in the presence of heparin/ACD and the screening cells for infection, wherein the presence of infection is indicative of particular HIV co-receptor usage.

Another aspect of the present invention provides a method for determining co-receptor usage of an HIV in a subject, the method comprising collecting blood for the subject in the presence of an anti-coagulant, generating plasma from the blood, culturing the plasma or the viral isolate derived from the plasma with cells which express surface CD4 and one or both of CCR5 and CXCR4 in presence of heparin/EDTA and the screening cells for infection, wherein the presence of infection is indicative of particular HIV co-receptor usage.

A further aspect of the present invention provides a method for determining co-receptor usage of an HIV in a subject, the method comprising collecting blood for the subject in the presence of an anti-coagulant, generating plasma from the blood, culturing the plasma or the viral isolate derived from the plasma with cells which express surface CD4 and one or both of CCR5 and CXCR4 in the presence of heparin/ACD/EDTA and the screening cells for infection wherein the presence of infection is indicative of particular HIV co-receptors usage.

The culturing step between the plasma or virus derived from the plasma and indicator cell lines may also be conducted in the presence of a positively charged polymer such as but not limited to polybrene (1,5-dimethyl-1, 5-diazaundecamethylene polymethobromide, hexadimethrine bromide) or SEVI. The present invention contemplates the use of spinoculation, an anti-coagulant and optionally a positively charged polymer or protein/s capable of enhancing infectivity.

Useful indicator cell lines include GHOSTX4R5 cells which are adherent cells from human osteocarcinoma cells and TZMb1 cells which are a HeLa cell line, but also include primary cells or cell lines which express CD4, CCR5 and CXCR4. Examples of additional indicator cell lines include BF24, CEM-GFP, GHOST Hi-5, GHOST CXCR4, GHOST X4/R5, HL3T1, LuSIV, M311, MT-4, T2M-bl, U87.CD4.CCR5, U373-MAGI, U373-MAGI-CXCR4, U373-MAGI-CCR5e, Sup-T1, Sup-T1-CCR5, U1/HIV-1, U937, MT-2, PMI, Cf2-Th-CD4, Cf2-Th-CD4-CCR5, Cf2-Th-CD4-CXCR4, MOLT-4, MOLT-4/CCR5 and MOCHA. Details of these example indicator cell lines can be found in Table 11.

**TABLE 11**

| **Cell line name** | **NIH cat#** | **Parental line** | **Species** | **CD4?** | **endogenous** | **transduced** | **type** | **promoter** |
|---|---|---|---|---|---|---|---|---|
| BF24 | 1296 | THP-1 (monocyte) | Human | Y | CXCR4(low), CCR5(PMA induced) | | CAT | HIV-1 LTR |
| CEM-GFP | 3655 | CEM | Human | Y | CXCR4 | | GFP | HIV LTR |
| GHOST Hi-5 | 3944 | HOS | Human | Y | CXCR4 | CCR5 | hGFP | HIV-2 LTR |
| GHOST CXCR4 | 3685 | HOS | Human | Y | CXCR4 | CXCR4 | hGFP | HIV-2 LTR |
| GHOST X4/R5 | 3942 | HOS | Human | Y | CXCR4 | CXCR4/CCR5 | hGFP | HIV-2 LTR |
| HL3T1 | 1115 | HeLa | Human | Y | CXCR4 | | CAT | HIV-1 LTR |
| LuSIV | 5460 | CEMx174 | Human | Y | CXCR4 | | Luciferase | SIVmac239LTR |
| M311 | 1295 | Molt-4 | Human | Y | CXCR4 | | CAT | HIV-1 LTR |
| MT-4 | 120 | (T-cell leukemia) | Human | Y | CXCR4 | | n/a | n/a |
| TZM-bl | 8129 | HeLa | Human | Y | CXCR4 | CCR5 | b-Gal, luciferase | HIV-1 |
| U87.CD4.CCR5 | 4035 | U87MG | Human | Y | | CCR5 | n/a | n/a |
| U373-MAGI | 3595 | U373MG | Human | Y | | | b-Gal | HIV-1 LTR |
| U373-MAGI-CXCR4 | 3596 | U373MG | Human | Y | | CXCR4 | b-Gal | HIV-1 LTR |
| U373-MAGI-CCR5e | 3597 | U373MG | Human | Y | | CCR5 | b-Gal | HIV-1 LTR |
| Sup-T1 | 100 | (T cell lymphoma) | Human | Y | CXCR4 | | n/a | n/a |
| Sup-T1-CCR5 | | (T cell lymphoma) | Human | Y | CXCR4 | CCR5 | n/a | n/a |
| U1/HIV-1 | 165 | U937 (HIV-1 infected) | Human | low | CXCR4, CCR5 | | n/a | n/a |
| U937 | | | Human | low | CXCR4, CCR5 | | n/a | n/a |
| MT-2 | 237 | (T cell leukemia) | Human | Y | CXCR4, CCR5 (low) | | n/a | n/a |
| PM1 | 3038 | HUT 78 | Human | Y | CXCR4, CCR5 | | n/a | n/a |
| Cf2-Th-CD4 | | (Thymocyte) | Canine | Y | | | n/a | n/a |
| Cf2-Th-CD4-CCR5 | | (Thymocyte) | Canine | Y | | CCR5 | n/a | n/a |
| Cf2-Th-CD4-CXCR4 | | (Thymocyte) | Canine | Y | | CXCR4 | n/a | n/a |
| MOLT-4 | 175 | - | Human | Y | CXCR4 | | n/a | n/a |
| MOLT-4/CCR5 | 4984 | Molt-4 | Human | Y | CXCR4 | CCR5 | n/a | n/a |
| MOCHA | | Molt-4 | Human | Y | CXCR4 | CCR5 | SEAP | HIV-1 LTR |

A kit is further contemplated herein comprising compartments adapted to contain reagents or samples such *inter alia* plasma, whole blood or indicator cell lines. The kit may be packaged for sale with instructions including generating plasma or culturing the plasma or virus derived from the plasma with indicator cell lines in the presence of an anti-coagulant such as heparin, ACD and/or EDTA.

The assay of this aspect of the present invention is useful in a therapeutic protocol.

As discussed herein a method for treating a subject infected with HIV may comprise determining co-receptor usage of the HIV in the subject by the method comprising culturing plasma or viral isolates from the plasma of a subject with cells which express surface CD4 and one or both of CCR5 and CXCR4 in the presence of an anti-coagulant and then screening cells for infection; wherein if infection occurs, administering to the subject an effective amount of a CCR5 antagonist or CXCR4 antagonist or a CD4/CCR5 or CD4/CXCR4 antagonist.

Again, useful anti-coagulants include heparin, ACD and/or EDTA.

As described herein the assay of the present invention may also be used to determine viral loads and such an assay forms part of the present invention.

Accordingly, another of the present invention contemplates a method for determining viral load in a subject, the method comprising culturing plasma or viral isolates derived from the plasma of the subject with cells capable of being infected by the virus, adding an anti-coagulant to the virus/cell culture, screening for infection and then determining viral genome copy number in infected cells or culture supernatant.

In a related embodiment, the present invention encompasses a method for determining viral load in a subject, the method comprising collecting blood from the subject in the presence of an anti-coagulant, generating plasma from the blood, preparing a viral isolate from the plasma, culturing the viral isolate with cells capable of being infected by the virus, adding an anti-coagulant to the viral isolate/cell culture, screening cells for infection and then determining viral genome copy number in infected cells.

The anti-coagulants may be heparin, ACD or EDTA or a combination thereof such as heparin/ACD, heparin/EDTA, ACD/EDTA or heparin/ACD/EDTA.

Determining infection of the indicator cells is conveniently accomplished using any method which allows for the detection of viral infection. Such methods include, without being limited to, the detection of reporter genes, such as fluorescent genes using for example, fluorescence microscopy, flow cytometry or the like. Alternatively, infection of the indicator cell lines may be detected using PCR or RT-PCR for viral genes or by detecting viral proteins using, for example, ELISAs, Western blots or the like. Viral genome copy number is generally determined genetically such as *via* an amplification reaction (e.g. RT-PCR) or by ELISA for viral proteins. Screening for viral infection may also be performed by using cell counts.

The determination of viral loads is useful *inter alia* for monitoring infection and/or monitoring the effectiveness of a treatment regime.

The determination of viral loads may be with respect to HIV or any other virus.

A rapid assay to detect HIV co-receptor usage from patient plasma without the need for prior amplification is described. The classification of co-receptor usage uses cell lines that selectively express each co-receptor. The major advance using this approach is speed and ease of technique.

The present invention is further described by the following non-limiting Examples. In the Examples the following methods are employed.

Blood is collected from HIV-1-infected individuals in sodium heparin, anti-coagulant citrate dextrose (ACD) a ethylenediamine tetraacetic acid (EDTA) and/or any other anti-coagulant blood tubes. The plasma isolated from each tube is stored at -80°C until use.

To pellet the virus, plasma was diluted 1 in 2 (to a final volume of 800µL) and layered over a 20% w/v sucrose cushion. The plasma was centrifuged at 17,000 x g for 2 hours at 4°C. The supernatant was aspirated and the viral pellet (viral isolate) was resuspended in 50µL of GHOST media (Dulbecco's Modified Eagle's Medium (DMEM, Invitrogen, Auckland, NZ), 10% v/v Cosmic calf serum (Quantum Scientific, Milton, Qld), 1× Penicillin/Streptomycin/Glutamine (Invitrogen), 1 µg/mL Puromycin (Sigma, St. Louis, MO), 100 µg/mL Hygromycin (Sigma), 500 µg/mL G418 (AG Scientific, San Diego, CA)) or DMEM-10 (DMEM, 10% v/v Cosmic calf serum) in the presence and absence of Heparin (20 U/mL, David Bull Laboratories, Melbourne, Australia). All samples contain polybrene at 2µg/mL. Samples resuspended in DMEM-10 contain 40 µg/mL DEAE-Dextran.

Samples were resuspended in GHOST media and added to GHOSTX4R5 cells which have been seeded 24 hours previously in a 96-well flat-bottomed plate at 10,000 cells/well. GHOSTX4R5 cells are an adherent cell line derived from human osteosarcoma (HOS cells). They are stably transfected with the HIV-2 LTR driving hGFP construct and express green fluorescent protein (GFP) when productively infected with HIV or SIV. The cells also stably express surface CD4, CCR5 and CXCR4 (Morner et al. Journal of Virology 73:2342 - 2349, 1999) (NIH AIDS Research and Reference Reagent Program, Division of AIDS, NIAID, NIH).

Samples are also resuspended in DMEM-10 media and added to TZMbl cells. TZMbl cells are a Hela cell which express CD4, CCR5 and CXCR4. These cells also stably transfected with β-galactosidase and luciferase under the control of the HIV-1 promoter. Upon integration of the host genome with the HIV-1 genome, these two genes are expressed and can be measured (NIH AIDS Research and Reference Reagent Program, Division of AIDS, NIAID, NIH *(*Derdeyn et al. Journal of Virology 74:8358-8367, 2000; Platt et al. Journal of Virology 72:2855-2864, 1998; Wei et al. Antimicrobial Agents and Chemotherapy 46:1896-1905, 2002). Following HIV infection of TZMbls, there is an increase in β-gal expression over the next 5 days. Infection is most simply measured by counting the number of cells that express β-gal (or are blue). The maximum number of blue cells that can be counted by this assay is 300 cells.

Both cell lines are infected at the same time with HIV-1 strains 89.6 (uses both CXCR4 and CCR5), NL4-3 (uses CXCR4 only) and AD8 (uses CCR5 only) as positive controls for infection. The cells are then subjected to spinoculation - centrifugation at 1200 x g for 2 hours at room temperature. The volume of media is increased to 150µL with media and the cells are incubated for 5 days. Samples are observed for the presence of clot formation at all stages.

At day 5 post-infection, GHOST cells are washed in phosphate buffered saline (PBS), detached from the plate with Trypsin-EDTA, washed twice in FACS wash and fixed with FACS Fix. The cells are then analyzed by flow cytometry for expression of GFP as an indication of HIV-1 infection. TZMbl cells are washed in PBS and fixed for 5 mins in fixative. Cells are washed once and 50µL of β-gal substrate is added to each well. The cells are incubated at 37°C for 2 hours. The substrate is removed and the cells are washed with and resuspended in PBS. Under a light microscope, the number of blue-stained (HIV-1 infected) foci are counted. Uninfected cells are analyzed as a negative/background control.

The time of plasma processing and storage temperature requirements were determined by collecting blood in two identical ACD tubes. Plasma was isolated from one tube at 1 hour after venepuncture, and the second tube was left on the bench until 4 hours after venepuncture before processing. Aliquots of plasma were then stored immediately after isolation at 4°C, -20°C and -80°C for 2 days. After 2 days in storage, the plasma samples were used to infect GHOSTX4/R5 cells.

To establish the influence of plasma pelleting time and speed, duplicate frozen plasma samples (from blood collected in ACD tubes) were diluted 1 in 2, in PD deficient in Ca²⁺ and Mg²⁺ (PBS) to a final volume of 800µL. 800µl was layered over 20% sucrose cushion (200µl) and centrifuged at 4°C for either 2 hours at 17,000 × g or 1 hour at 45,000 × g. The viral pellets were resuspended and used to infect GHOST X4R5 cells. The results for the samples prepared under the two different conditions were compared. All plasma samples were processed within 8 hours from the time of collection

The seeding time and condition of test cells was determined to elucidate whether the time of the assay could be reduced. Prior to infection, GHOSTX4R5 cells were removed from the culture flasks with EDTA (2.5 µM) and seeded at 10,000 cells per well into 96-well flat-bottomed tissue culture plates for either 1 or 2 days prior to infection. Cells were inoculated with duplicate samples of frozen plasma and the results from the different seeding conditions were compared.

Optimal spinoculation temperature analysed when GHOSTX4R5 cells were seeded for 48 hours and then infected with plasma samples that had been pelleted at 17,000 × g for 2 hours and resuspended in GHOST media containing heparin (20 U/ml) and polybrene (2.5 µg/ml). Duplicate plates were prepared. One plate was subjected to spinoculation (centrifugation at 1,200 × g) at 37°C for 2 hours, while the other plate was spun at the same speed and time but at approximately 20°C. At day 6 post-infection, the results from the two conditions were compared.

To resolve whether the use of semen enhancing viral infectivity (SEVI) peptides could enhance the sensitivity of detection of the virus from patient plasma, SEVI peptide fragments were suspended in PD (2mg of lyophilized peptide in a volume of 200µL) and agitated at 250rpm overnight at 37°C to generate fibrils as previously described (Munch et al., Cell: Dec 2007). GHOSTX4 or GHOSTX4/R5 and Tzmbl cells were seeded at a density of 4000 cells per well in a 96 well plate overnight. The following day cells were infected with different cpm/cell of NL4.3 (X4 using) or YU2 (R5 using) virus in the presence and absence of SEVI fibril solution (50µg/ml). The number of GFP positive cells (GHOSTX4 or GHOSTX4/R5) or number of blue cells (Tzmbl) was compared as a measure of infectivity.

To establish optimal assay sensitivity the time of infection was prolonged. GHOSTX4R5 cells were inoculated with duplicate plasma samples (inoculation volume 50µL) and subjected to spinoculation. In one of the duplicate samples, 100µL of GHOST media was added to each well immediately after spinoculation (as usually done in the current protocol). In the other wells, additional media was added >12 hours after spinoculation, during which time the cells had been maintained in the incubator. The number of GFP positive cells using each set of conditions was compared.

The best volume of patient plasma which improved assay sensitivity was determined by collecting plasma in ACD tubes. The plasma was then isolated within 4-24 hours and pelleted from a starting volume of plasma of either 400µl, 800µL or 1.6mL from the same patient and then infected GHOSTX4R5 cells.

### EXAMPLE 1

### Heparin inhibits the formation of clots when patient plasma is added directly to cell culture media and cells

The results are shown in Figure 1(a).

Of the patient samples prepared in DMEM-10 (n = 8), clotting was reduced when blood was collected in ACD (87.5%) or EDTA (62.5%), without the addition of heparin. Clotting was observed in fewer samples derived from blood collected in sodium heparin (25%). When heparin was added to samples after collection in ACD or EDTA, no clotting was observed.

Of the patient samples prepared in GHOST media (n = 14), no clotting was observed in samples derived from blood collected in sodium heparin. Some clotting was observed in samples derived from blood collected in ACD (79%) and EDTA (100%), without the addition of heparin. When heparin was added to samples collected in ACD or EDTA, no clotting was observed.

The data presented in this figure are generated from the protocol as stated, however, storage time and temperature of storage varied between 2 and 24 hours. In addition, some patient samples were frozen at -80°C while others were processed immediately. Infection was detected using microscopy as appropriate per indicator cell line used.

### EXAMPLE 2

### The addition of heparin to viral isolate derived from plasma from blood collected in ACD had the highest sensitivity for detection of virus

The results are shown in Figure 1(b).

Of the patient samples prepared in DMEM-10 (n = 9) and used to inoculate TZMbl cells, viral infection was most frequently observed when the plasma had been collected in heparin (33%), compared with 22% of samples collected in either ACD or EDTA.

Of the patient samples prepared in GHOST media (n = 10) and used to inoculate GHOSTX4R5 cells, viral infection was most frequently observed when the plasma had been collected in ACD, and heparin had been added to the resuspended plasma pellet (50%). Viral infection was observed in 40% of samples collected in heparin and ACD, as well as samples collected in EDTA to which heparin had been added. Only 30% of samples collected in EDTA were positive for productive infection.

The data presented in this figure are generated from the protocol as stated, however, storage time and temperature of storage varied between 2 and 24 hours. In addition, some patient samples were frozen at -80°C while others were processed immediately. Infection was detected using microscopy as appropriate per indicator cell line used.

### EXAMPLE 3

### Sensitivity of assay for detection of virus from plasma using indicator cell lines

Patient plasma was collected and cells infected as described above. TZMbl cells were infected with viral isolates from plasma from blood collected in heparin tubes, whilst GHOST cells were infected with plasma virus derived from blood collected in ACD tubes. Heparin was then added (20U/mL) to the viral isolate pellet.

TZMbl cells were infected with viral isolates from either 400µL or 800µL of plasma and infection was detected at day 5 post-infection by staining with β-gal stain as described above. Cells positive for HIV-1 infection were counted under a light microscope at 10× magnification.

GHOSTX4R5 cells were infected with viral isolates derived from either 800µL or 1.6mL of plasma and cells positive for HIV-1 infection were counted at day 7 post-infection under fluorescence microscopy in the PC3.

Samples were collected from patients with viral loads (as determined by RT-PCR, Roche Cobas assay, Roche Diagnostics, Basel, Switzerland) ranging from undetectable (< 50 copies/mL) to >100,000 copies/mL (n = 50).

Using the GHOSTX4R5 cell line (Figure 2), detection of infection from 800µL of plasma by fluorescence microscopy yielded the most consistent and sensitive results (n = 36; median (range) viral load was 10,000 (<50 to >100,000) copies/mL). Virus was consistently detected from patients with viral loads > 20,000 copies/mL. The median (range) of the patient viral load where a sample was positive using this assay was 18,300 (<50 to >100,000) copies/ml.

Using the TZMbl cell line (Figure 2), a plasma volume of either 400µL or 800µL was used as there was little difference in sensitivity with either volume (n = 40, median (range) viral load 5,850 (<50 to >100,000) copies/mL). Virus was consistently detected from patients with viral loads > 70,000 copies/mL. The median (range) of the patient viral load where a sample was positive using this assay was 73,000 (<50 to >100,000) copies/ml.

For patients with undetectable viral loads (<50 copies/ml), there were no positive results using either cell line.

Therefore, nearly all patients with a plasma HIV VL>20,000 and 70,000 copies/ml had detectable virus using GHOSTX4R5 and TZMB1 cells respectively.

The data presented in this figure are generated from the protocol as stated, however, storage time and temperature of storage varied between 2 and 24 hours. In addition, some patient samples were frozen at -80°C while others were processed immediately. Infection was detected using microscopy as appropriate per indicator cell line used.

### EXAMPLE 4

### Determining the tropism of patient plasma virus in indicator cell lines

### Bench Marking Methodology

Patient virus which had been amplified from either plasma or PBMC and co- cultured with donor PBMC was used to inoculate GHOST cells expressing either CXCR4 or CCR5. The co-receptor usage of these viral isolates was compared to a previously validated assay using the Cf2-luc indicator cell line, Gorry et al. Journal of Virology 75:10073-10089, 2001. On day 7 post-infection, the cells were assessed by fluorescence microscopy for the presence of infected cells. Samples that were able to infect CCR5-expressing cells were designated R5-tropic, and samples which were able to infect CXCR4-expressing cells were designated X4-tropic.

Viral isolate derived from the patient plasma were collected and GHOST cells expressing either CXCR4 or CCR5 were infected as described above. On day 7 post-infection, the cells were assessed by fluorescence microscopy for the presence of infected cells and tropism was assigned to each sample.

A total of 18 viral isolates amplified/collected from either patient plasma or peripheral blood mononuclear cells were collected from 13 patients and tested by both assays. All samples were positive for infection using GHOSTR5X4 cells. Of the 18 samples tested, only 3 results differed from the previous assay conducted in Cf2-luc cells. In 2 of the three cases, minor CCR5 usage as characterized by the amplification methodology was not observed with the invention assay. In the third case, a virus which had been previously characterized as dual tropic was characterized as CCR5-using in the GHOST cell assay (Table 1). Results in brackets denotes minor usage; X4 = CXCR4-using; R5 = CCR5-using.

**TABLE 1**

| Co-receptor usage of virus amplified from either patient plasma or peripheral blood mononuclear cells (PBMC), where '(R5)' and '(R4)' indicates minor usage. | | |
|---|---|---|
| **PATIENT ID** | **CF2-luc Assay ("Bench Mark Method")** | **An example of the Method of the present invention** |
| 01 PBMC | R5 X4 | R5 |
| 01 PLASMA | R5 | R5 |
| 02 PBMC | R5 | R5 |
| 02 PLASMA | R5 | R5 |
| 03 PBMC | R5 | R5 |
| 03 PLASMA | X4 (R5) | X4 R5 |
| 04 PLASMA | R5 | R5 |
| 05 PBMC | X4 (R5) | X4 |
| 05 PLASMA | R5 | R5 |
| 07 PBMC | R5 X4 | R5 X4 |
| 07 PLASMA | R5 (X4) | R5 |
| 08 PBMC | R5 | R5 |
| 09 PBMC | R5 | R5 |
| 10 PBMC | R5 | R5 |
| 11 PBMC | X4 R5 | X4 R5 |
| 12 PBMC | X4 R5 | X4 R5 |
| 13 PLASMA | R5 | R5 |
| 14 TISSUE | R5 | R5 |

The data presented in this figure are generated from the protocol as stated, however, storage time and temperature of storage varied between 2 and 24 hours. In addition, some patient samples were frozen at -80°C while others were processed immediately. Infection was detected using microscopy as appropriate per indicator cell line used.

Nine plasma samples (collected in ACD+heparin) were then assessed for co-receptor usage using the GHOSTR5 and GHOSTX4 cell lines. All 9 virus samples used CCR5. Six samples were also able to infect the GHOST cells using CXCR4. (Table 2).

**TABLE 2**

| Co-receptor usage of HIV-1 from patient plasma without amplification. HAART = highly active antiretroviral therapy. Y=yes, N=no. | | | |
|---|---|---|---|
| **PATIENT ID** | **GHOST cell Assay** | **VIRAL LOAD Copies/ml** | **On HAART?** |
| 2S | R5 X4 | > 100 000 | Y |
| 2X | R5 X4 | > 100 000 | Y |
| 3F | R5 | 98 400 | N |
| 3Q | R5 X4 | 90 900 | N |
| 2H | R5 X4 | 87 600 | N |
| 2M | R5 X4 | 58 600 | N |
| 3U | R5 | 36 700 | Y |
| 3J | R5 | 20 500 | N |
| 3T | R5 X4 | 16 700 | N |

The data presented in this figure are generated from the protocol as stated, however, storage time and temperature of storage varied between 2 and 24 hours. In addition, some patient samples were frozen at -80°C while others were processed immediately. Infection was detected using microscopy as appropriate per indicator cell line used.

### EXAMPLE 5

### Infection of ghostX4R5 indicator cell line with hiv-1 patient sera - determining the effect of anti-coagulants on clotting, sensitivity and efficiency of infection

Matched patient blood samples were collected from HIV-1 positive, viraemic individuals, in sodium heparin, ACD and EDTA blood tubes.

To prepare a viral isolate pellet, plasma was diluted 1 in 2 (to a final volume of 800µL) and layered over a 20% v/v sucrose cushion. The plasma was centrifuged at 17,000 x g for 2 hours at 4°C. The supernatant was aspirated and the viral pellet was resuspended in 50µL media as follows:
Sodium heparin samples:
   Resuspended in GHOST media.
ACD samples:
   One sample resuspended in GHOST media.
   One sample resuspended in GHOST media plus Heparin at 20U/mL.
   One sample resuspended in GHOST media plus EDTA*
EDTA samples:
   One sample resuspended in GHOST media.
   One sample resuspended in GHOST media plus Heparin at 20U/mL.
   One sample resuspended in GHOST media plus ACD*
      *ACD and EDTA were obtained by filling collection tubes with 9mL of GHOST media. This media was then used undiluted to resuspend the pellet.

All samples contained Polybrene at 2µg/mL.

Samples were added to GHOSTX4R5 cells which had been seeded the night before in a 96-well flat-bottomed TC plate at 10,000 cells/well. The cells were subjected to Spinoculation - centrifugation at 1200 x g for 2 hours at room temperature. The volume of media was increased to 150µL with GHOSTX4R5 media and the cells were incubated for 5 days. Samples were observed for the presence of clot formation at all stages.

The cells were washed in PD, detached from the plate with Trypsin-EDTA, washed 2X in FACS wash and fixed with FACS Fix. The cells were then analyzed by flow cytometry for GFP expression as an indication of HIV-1 infection. Cells infected with HIV-1 lab strains 89.6, NL4-3 and AD8 were run concurrently as positive controls for infection. Uninfected cells were analyzed as a negative/background control.

Some clotting was observed when virus pelleted from ACD or EDTA plasma was added to cell culture media prior to the addition of cells in samples from 5 out of 5 patients.

Some clotting was observed in wells containing cells which had been infected with pelleted virus collected in ACD or EDTA, to which had been added EDTA or ACD, respectively, at Day 5 post-infection.

No clotting was observed at any time point when samples were derived from blood collected in sodium heparin, or in samples derived from ACD or EDTA blood, to which heparin had been added after the virus had been pelleted.

Accordingly, heparin is particularly useful inhibiting the formation of clots. The addition of Heparin to samples derived from blood collected in ACD or EDTA inhibits clotting. The results are shown in Figure 3.

In samples from 5 patients tested, viral isolates derived from ACD plasma, and to which heparin had been added, was observed to infect GHOSTX4R5 cells.

Of the 5 patients, virus from the following number of patients were observed to infect GHOSTX4R5 cells when prepared with the following remaining anti-coagulant combinations:
Sodium heparin = 3
ACD = 3
ACD + EDTA = 1
EDTA = 4
EDTA + Heparin = 4
EDTA + ACD = 3

Hence, virus derived from ACD plasma, to which heparin has been added, is most likely to be able to infect GHOSTX4R5 cells to detectable levels by Day 5 post-infection. The results are shown in Figure 4.

In samples from 5 patients tested, the mean number of GFP positive cells per 100,000 cells was calculated for each anti-coagulant combination. The mean was highest for samples derived from blood collected in sodium heparin. Addition of heparin to samples derived from blood collected in either ACD or EDTA slightly improved the mean number of infected cells over ACD or EDTA alone. When ACD or EDTA was added to samples, the mean number of infected cells was very low. The means include samples where no infection was observed.

Accordingly, when infection is observed, a higher proportion of cells will be infected by virus derived from blood collected in sodium heparin. The results are shown in Figure 5.

### EXAMPLE 6

### Infectivity is reduced if plasma is processed later than 4 hours after collection.

Results are summarised in Table 3 and Figure 6.

For the first 6 samples, processing plasma within 1 and 4 hours as well as storage temperature of 4°C, -20°C and -80°C was examined. The infectivity of samples stored at 4°C was significantly lower compared to storage at -20°C and -80°C. Hence for next 6 samples only -20°C and -80°C storage temperatures were examined.

The median (range) VL was 550 (50 - 5690 copies/ml; n=12). For samples stored at - 20°C, 10/12 and 8/12 were positive when plasma was stored within 1 or 4 hours respectively (p=0.500). For samples stored at -80°C, 10/12 and 8/12 were positive when plasma was stored within 1 or 4 hours respectively (p=0.812). Therefore there was no significant difference in the number of positive samples if plasma was processed within 1 or 4 hours.

When comparing the number of GFP positive cells as a continuous variable, there was also no statistically significant difference between processing within 1hr or 4 hrs. When blood was processed at 1 hr there was no statistically significant difference between the samples stored at either -20°C or -80°C. However when the plasma was processed at 4hrs, the number of infected cells was significantly lower if the storage temperature was -20°C when compared to storage at -80°C (p=0.047).

In other words the sensitivity of the assay was decreased when the samples were processed after 4hr and stored at -20°C.

**TABLE 3**

| The effect of temperature variation infectivity. VL = viral load; ND = not done, NR = No Result. | | | | | | | |
|---|---|---|---|---|---|---|---|
| | | **1 hour** | | | **4 hours** | | |
| **INITIALS** | **VL Copies/ml** | **4°C** | **-20°C** | **-80°C** | **4°C** | **-20°C** | **-80°C** |
| GAMA | 56900 | <2 | 64 | 58 | 8 | 48 | 72 |
| SCED2 | 5400 | 7 | 12 | 10 | <2 | <2 | <2 |
| GOTH | 5250 | 7 | <2 | <2 | <2 | <2 | <2 |
| NOGE | 600 | <2 | <2 | <2 | <2 | <2 | <2 |
| BUD | 500 | <2 | >300 | >300 | <2 | <2 | 3 |
| DEWI2 | 50 | <2 | <2 | <2 | <2 | <2 | <2 |
| IVBA | 15600 | ND | 10 | 9 | ND | 7 | 19 |
| ROMO | 3900 | ND | 14 | 40 | ND | 17 | 31 |
| GLPA | 500 | ND | 11 | 18 | ND | 11 | 18 |
| GAMA | 150 | ND | 5 | 13 | ND | 7 | 6 |
| ERWA | 50 | ND | 5 | 13 | ND | 12 | 21 |
| ROMI | 50 | ND | 5 | 7 | ND | 8 | 8 |
| +ve control | ---- | | NR | NR | | NR | NR |
| -ve control | ---- | | <2 | <2 | | <2 | <2 |
| **Median** | **550** | **2.0** | **7.5** | **11.5** | **2.0** | **7.0** | **7.0** |
| **IQR** | **75-5363** | **2-7** | **2.75-13.50** | **3.25-34.50** | **2.0-3.5** | **2.0-11.75** | **2.0-20.50** |

### EXAMPLE 7

### Increase in spinoculation speed and reduction in time has no effect on sensitivity.

The data is summarised in Table 4 and Figure 7.

The median (range) VL for the samples was 32 950 (250-100 000) copies/ml (n=14).. All samples were positive when plasma was centrifuged for either 1 or 2 hours. When comparing the number of GFP positive cells as a continuous variable, there was no statistically significant difference between the number of GFP positive cells when prepared with different centrifuge speeds and duration.

**TABLE 4**

| The effect of spinoculation speed and time on infectivity. | | | |
|---|---|---|---|
| | | **# pos cells** | |
| **INITIALS** | **VL Copies/ml** | **17000g/2hrs** | **45000g/1hr** |
| GUAL | >100000 | >300 | >300 |
| VAST | >100000 | >300 | >300 |
| LUTI | 72200 | 154 | 87 |
| MAGA | 56900 | 54 | 78 |
| KIRK | 55300 | >300 | >300 |
| LOHO | 47600 | 193 | 123 |
| LOHO | 47600 | 91 | 56 |
| GRRO | 18300 | 8 | 35 |
| SCED | 5400 | 57 | 112 |
| DAPO | 2900 | 38 | 58 |
| ERWA2 | 1700 | 48 | 82 |
| MOEL | 1500 | 30 | 30 |
| NOGE | 600 | 150 | 150 |
| WAMO | 250 | 32 | 29 |
| +ve control | ---- | >300 | >300 |
| -ve control | ---- | <2 | <2 |
| **Median** | **32950** | **74** | **85** |
| **IQR** | **1650-60725** | **37-220** | **51-188** |

### EXAMPLE 8

### Cells seeded 48 hours prior to infection improved infectivity

The data is summarised in Table 5 and Figure 8.

The median (range) VL for the samples assessed was 72 200 (250->100000) copies/ml (n=11). 8/11 and 9/11 were positive when cells were seeded for either 1 or 2 days prior to infection respectively.

When comparing the number of GFP positive cells as a continuous variable, there was a significant increase in the number of GFP cells when cells were seeded for 48 hours compared to 24 hours (p=0.008). Linear regression analysis suggested a significant association between viral load and number of infected cells detected only when cells were seeded at 48hrs.

**TABLE 5**

| The effect of test cell seeding time on infectivity. | | | |
|---|---|---|---|
| | | **# pos cells** | |
| **INITIALS** | **VL** | **24hrs** | **48hrs** |
| VAST | >100000 | >300 | >300 |
| DUSM | >100000 | 3 | 26 |
| MATI3 | >100000 | 12 | 42 |
| BEMI | >100000 | <2 | <2 |
| MATI2 | >100000 | 34 | 81 |
| LUTI | 72200 | 3 | 14 |
| MAGA | 56900 | 3 | 10 |
| HRL | 55500 | 8 | 20 |
| IB2 | 34700 | 6 | 12 |
| SGR | 7900 | <2 | 3 |
| WHST2 | 250 | <2 | <2 |
| +ve control | ------- | >300 | >300 |
| -ve control | ------- | <2 | <2 |
| **Median** | **72200** | **3.0** | **14.0** |
| **IQR** | **34700-100000** | **2.0-12.0** | **3.0-42.0** |

### EXAMPLE 9

### Spinoculation temperature does not effect infectivity.

The results are as shown in Table 6 and Figure 9.

The median (range) VL for the samples assessed was 98 400 (7000 ->100,000 copies/ml) (n=7), 7/7 and 5/7 were positive for infection when spinoculation was performed at 20°C and 37°C respectively.

**TABLE 6**

| The effect of spinoculation temperature on infectivity. | | | |
|---|---|---|---|
| | | **#pos cells** | |
| **INITIALS** | **VL Copies /ml** | **20°C** | **37°C** |
| FACH | >100000 | <2 | <2 |
| GUAL | >100000 | 30 | 42 |
| WIRO | >100000 | <2 | <2 |
| PEDI | 98400 | 3 | <2 |
| KIRY | 55300 | 17 | 5 |
| NAEL | 20400 | 200 | 100 |
| GRRO | 7000 | 100 | 100 |
| +ve control | -------- | >300 | >300 |
| -ve control | -------- | <2 | <2 |
| **Median** | **98400** | **17** | **5** |
| **IQR** | **20400-100000** | **2-100** | **0-100** |

### EXAMPLE 10

### SEVI peptide increases the sensitivity of NL4.3 virus detection.

Results are summarized in Table 7 & 8 and Figure 10 & 11.

The GHOSTX4, GHOSTX4/R5 and Tzmbl cells were infected with NL4.3 or YU2 at 10.0, 1.0, 0.1 and 0.0 cpm/cell in the presence and absence of SEVI fibril solution (50µg/ml). For infection with NL4.3 virus a significant increase in the number of blue cells (Tzmbl's) and GFP positive cells (GHOST X4) were observed in samples containing SEVI (p<0.001). However, for infection with YU2 no increase in the number of blue cells (Tzmbl's) and GFP positive cells (GHOSTX4R5) was observed in samples containing SEVI. Therefore, addition of SEVI enhanced viral infection with NL4.3 virus but not YU2 virus.

**TABLE 7**

| Number of cell infected with NL4.3 virus at different MOIs in the presence of absence of SEVI peptide. | | | | |
|---|---|---|---|---|
| **cpm/cell** | **Tzmbl (# of Blue cells)** | | **GHOST X4 (# of GFP +ve cells)** | |
| | **+SEVI** | **-SEVI** | **+SEVI** | **-SEVI** |
| **10.0** | 300.0 ± 0.0*** | 21.8 ± 12.1 | 67.0 ± 0.5*** | 5.3 ± 0.8 |
| **1.0** | 97.3 ± 7.6*** | 1.3 ± 0.95 | 17.3 ± 0.3*** | 1.5 ± 1.5 |
| **0.1** | 8.8 ± 3.0 | 0.5 ± 0.3 | 8.8 ± 2.3* | 1.8 ± 1.8 |
| **0.0** | 0.0 ± 0.0 | 0.0 ± 0.0 | 0.0 ± 0.0 | 0.0 ± 0.0 |

**TABLE 8**

| Number of cell infected with YU virus at different MOIs in the presence of absence of SEVI peptide. | | | | |
|---|---|---|---|---|
| **cpm/cell** | **Tzmbl** | | **GHOST X4/R5** | |
| | **+SEVI** | **-SEVI** | **+SEVI** | **-SEVI** |
| **10.0** | 300.0 ±0.0 | 300.0 ± 0.0 | 300.0 ± 0.0 | 300.0 ± 0.0 |
| **1.0** | 264.0 ± 21.2 | 248.5 ± 29.7 | 300.0 ± 0.0 | 300.0 ± 0.0 |
| **0.1** | 59.3 ± 10.1 | 58.5 ± 13.0 | 150.0 ± 0.0 | 150.0 ± 0.0 |
| **0.0** | 0.0 ± 0.0 | 0.0 ± 0.0 | 0.0 ± 0.0 | 0.0 ± 0.0 |

### EXAMPLE 11

### Overnight incubation does not improve infectivity.

The results are shown in Figure 12 and Table 9.

The median (range) VL for the samples assessed was 45100 (250->100000). Overnight incubation of the indicator cell lines with the inoculated viral isolate did not significantly increase the sensitivity of the assay.

**TABLE 9**

| Numbers of infected cells after spinoculation or overnight incubation. | | | |
|---|---|---|---|
| | | **# pos cells** | |
| **INITIALS** | **VL Copies/ml** | **NORMAL** | **OVERNIGHT** |
| BEMI | >100000 | <2 | <2 |
| MATI2 | >100000 | 81 | 114 |
| WIRO | >100000 | <2 | <2 |
| HRL | 55500 | 20 | 4 |
| IB2 | 34700 | 12 | <2 |
| NAEL | 20400 | 200 | 150 |
| GRRO | 7000 | 100 | 100 |
| WHST2 | 250 | <2 | <2 |
| +ve control | ------ | >300 | >300 |
| -ve control | ------ | <2 | <2 |
| **Median** | **45100** | **16** | **3** |
| **IQR** | **10350-100000** | **2-95** | **2-111** |

### EXAMPLE 12

### Plasma volume does not affect assay sensitivity

The results are shown in Table 10.

The sensitivity of the assay was first compared when using either 400µl or 800µl of plasma. The median (range) VL for the samples assessed was 2900 (250 - >100,000 copies/ml; n=11). There was no significant difference in the sensitivity of detection of infectious virus between plasma volumes of 400µL and 800µL (Wilcoxon signed rank test (p = 0.66) and McNemar test (p = 1.00) see Figure 13).

**TABLE 10**

| Detected infection of GHOST X4R5 cells with plasma volumes of 0.4 or 0.8ml. | | | | |
|---|---|---|---|---|
| | | | **# pos cells** | |
| **Patient** | **Operator** | **VL Copies/ml** | **0.4mL confocal** | **0.8mL confocal** |
| GUAL | LS/AS | >100000 | 300 | 300 |
| MATI2 | AS/AC | >100000 | 67 | 81 |
| BEMI | AS/AC | >100000 | <2 | <2 |
| HRL | AS/AC | 55500 | 100 | 20 |
| SCED | LS/AS | 5400 | 58 | 57 |
| DAPO | LS/AS | 2900 | 23 | 38 |
| ERWA2 | LS/AS | 1700 | 53 | 48 |
| MOEL | LS/AS | 1500 | 37 | 30 |
| NOGE | LS/AS | 600 | 75 | 150 |
| WHST2 | LS/AS | 250 | <2 | <2 |
| WAMO | LS/AS | 250 | 20 | 32 |
| +ve control | LS/AS | ----- | >300 | >300 |
| -ve control | LS/AS | ----- | <2 | <2 |
| **Median** | | **2900** | **53** | **38** |
| **IQR** | | **600-100000** | **20-75** | **20-81** |

### EXAMPLE 13

### Determination of HIV infectivity

Approximately, 9ml whole blood is collected from HIV-1 infected individuals in vacuum tubes containing ACD-B (citric acid 0.88mg/ml; trisodium citrate 2.2mg/ml; glucose (dextrose) 2.45mg/ml). Within 2 hours of collection the plasma is processed as per Monogram's recommendations. To separate the plasma from cells, tubes are centrifuged at 1000xg for 10 minutes with no brakes at room temperature. Plasma is aliquoted into 1.5ml microfuge tubes and stored at -80°C for at least 24hours prior to use.

GHOST cells are seeded into 96 well plates at 10,000 cells/well in a volume of 100µl/well 48 hours prior to inoculation. The outside wells were not seeded but were filled with sterile PBS.

Plasma samples (800µl) were aliquoted from ACD tubes and diluted in PBS to 1.6ml. In a microfuge tube 800µl of diluted plasma was mixed with 200µl of 20% sucrose in PBS. The sample was pelleted by centrifugation at 4°C at 17,000g for 2 hours to isolate the virus. Without disturbing the pellet the liquid was removed and the pellet was resuspended in 25µl of GHOST media + 20U/ml heparin +2.5µg/ml polybrene with or without other compounds as required. Pairs of tubes were then consolidated to form a total volume of 50µl. Samples were then used to inoculate wells immediately.

Inoculation of the seeded wells occurred with the total 50µl of each sample. The plates were then sealed with paraffin or a zip lock bag to prevent contamination. Plates were then spinoculated for 2 hours at 1,200g at 20°C. Culture media was added to each inoculated well (100µl) and then plates were incubated for 6 days in 5% CO₂.

Final analysis of the infectivity was carried out by aspirating the media and replacing it with PD. Wells were then viewed under a fluorescence microscope with a 10x magnification using a FITC/GFP filter. The number of fluorescent green cells in each well was recorded.

### EXAMPLE 14

### Co-receptor usage as measured using PCR

Viral infection of the indicator cell lines was also determined using PCR analysis. Briefly, Cf₂th (no CD4 or CXCR4) and Cf₂X4 (CD4 and CXCR4) cells were seeded 24 hrs prior to infection. After 24 hrs, the cells were infected with the X4 laboratory virus (NL4.3) at 10 or 0 cpm/cell. At Day 3 and Day 7 cells were washed three times with PBS and the DNA lysates were prepared.

Cells were lysed in a lysis buffer containing: Tris pH 8.0 (final concentration of 10 µM), EDTA (1 µM), Triton₁₀₀/SDS (in H₂O; final concentration 0.002%) and proteinase K (final concentration 0.8mg/ml). The cells/buffer were then heated to 56°C for 60 minutes, followed by 95°C for 10 minutes.

PCR was then performed on the cellular lysate using the following primers:
Forward primer: 5' - TCTCTAGCAGTGGCGCCCGAACA (SEQ ID NO: 1);
Reverse primer: 5' - TCTCCTTCTAGCCTCCGCTAGTC - 3' (SEQ ID NO:2); and
HIV beacon: 5' (fam) cgggag tactcaccagtcgccgcccctcgcc ctcccg (Dabcyl) 3' (SEQ ID NO:3).

PCR amplification was performed using the following conditions for 45 cycles:
94°C for 20 seconds;
55°C for 40 seconds;
72°C for 40 seconds.

As shown in Figure 14, the lower detection limit of the assay is represented by the dotted line which is 10 copies.

Using the same methods, Cf₂th (no CD4 or CCR5) and Cf₂R5 (CD4 and CCR5) cells were seeded 24 hrs prior to infection. After 24 hrs cells were infected with an R5 virus (YU2) at 10 or 0 cpm/cell. At Day 3 and Day 7 cells were washed three times with PBS and the DNA lysates were prepared.

As shown in Figure 15, the lower detection limit of detection of the assay is represented by the dotted line is 10 copies.

Those skilled in the art will appreciate that the invention described herein is susceptible to variations and modifications other than those specifically described. It is to be understood that the invention includes all such variations and modifications which fall within the scope of the claims. The invention also includes all of the steps, features, compositions and compounds referred to, or indicated in this specification, individually or collectively, and any and all combinations of any two or more of said steps or features, all of which fall within the scope of the claims.

### BIBLIOGRAPHY

Cooley et al. J Clin Virol 26(2):121-132, 2003
Derdeyn et al. Journal of Virology 74: 8358-8367, 2000
Gorry et al. Journal of Virology 75:10073-10089, 2001
Morner et al. Journal of Virology 73:2342 - 2349, 1999
Platt et al. Journal of Virology 72:2855-2864, 1998
Solomon et al. Journal ofAcquired Immune Deficiency Syndrome 40:140-148, 2005
Solomon et al. Journal of Infectious Diseases 187:1915-1923, 2003
Wei et al. Antimicrobial Agents and Chemotherapy 46:1896-1905, 2002

## Claims

1. A method for determining co-receptor usage of an HIV in a subject, said method comprising culturing a viral preparation derived from said subject's plasma with one or more cell lines selected from:
(i) cells which express surface CD4 and CCR5;
(ii) cells which express surface CD4 and CXCR4; and
(iii) cells which express surface CD4, CCR5 and CXCR4, wherein an anti-coagulant is added to the viral preparation or to the culture;
and screening for infection wherein the presence or absence of infected cells is indicative of the co-receptors used by the HIV.

2. The method of claim 1 wherein the viral preparation is collected in the presence of an anti-coagulant which may be the same or different to the added anti-coagulant.

3. The method of claim 1 or 2 wherein the anti-coagulant is selected from an anti-coagulant, an anti-platelet drug, a thrombolytic or fibrinolytic drug and a non-medicinal agent.

4. The method of claim 3 wherein the anti-coagulant is heparin, citrate dextrose (ACD), EDTA, a combination of heparin and ACD or EDTA, or a combination of heparin, ACD and EDTA.

5. The method of any one of clams 1 to 4 wherein the plasma is derived from whole blood collected in the presence of an anti-coagulant.

6. The method of any one of claims 1 to 5 wherein the culturing step further comprises a cationic polymer.

7. The method of claim 6 wherein the cationic polymer is polybrene.

8. A method as claimed in any one of claims 1 to 7 wherein said method comprises collecting blood from said subject in the presence of an anti-coagulant, to generate plasma from the blood for use in the method.

9. A method of selecting a viral antagonist suitable for treating a subject infected with HIV, said method comprising determining co-receptor usage by the method of any one of Claims 1 to 8 and selecting a viral antagonist based on co-receptor usage.

10. A method for determining viral load in a subject, the method comprising culturing virus isolated from plasma from the subject with cells capable of being infected by the virus, adding an anti-coagulant to the virus/cell culture, screening for infection and then determining viral genome copy number in infected cells or culture supernatant.

11. The method of claim 10 wherein the virus is HIV.

12. The method of any one of claims 1 to 11 wherein the HIV is HIV-1.

13. The method of any one of claims 10 to 12 wherein the anti-coagulant is selected from an anti-coagulant, an anti-platelet drug, a thrombolytic or fibrinolytic drug and a non-medicinal agent.

14. The method of any one of claims 5 to 13 wherein the anti-coagulant is heparin, ACD and/or EDTA.

15. The method of claim 1, 8, 9 or 13 wherein the anti-coagulant is not a metal ion chelator.

16. The method of claim 1, 8, 9 or 13 wherein the anti-coagulant is not a metal ion chelator selected from EDTA, ACD and oxalate.

17. The method of any one of claims 10 to 16 wherein viral genome copy number is determined by RT-PCR.

18. Use of one or more anticoagulants and indicator cell lines expressing surface CD4 and one or both of CCR4 and CXCR5 in the manufacture of an assay to determine co-receptor usage of HIV or to determine viral load.

19. An assay kit for determining co-receptor usage of HIV or to determine viral load, wherein the kit comprises one or more anticoagulants and indicator cell lines expressing surface CD4 and one or more of CCR4 and CXCR5.

20. The method, use or assay kit of any one of claims 1 or 8 to 19 wherein the cell lines are selected from GHOSTX4R5 and TZMb1 cells.

21. A method as claimed in any one of Claims 1 to 17 using an assay kit as claimed in claim 19.

## Patentansprüche

1. Verfahren zum Bestimmen der Korezeptor-Verwendung eines HIV in einem Subjekt, wobei das Verfahren das Kultivieren eines Viruspräparats, das aus dem Plasma des Subjekts stammt, mit einer oder mehreren Zelllinien, die aus folgenden ausgewählt ist bzw. sind:
(i) Zellen, die Oberflächen-CD4 und CCR5 exprimieren;
(ii) Zellen, die Oberflächen-CD4 und CXCR4 exprimieren; und
(iii) Zellen, die Oberflächen-CD4, CCR5 und CXCR4 exprimieren, wobei ein Antikoagulans dem Viruspräparat oder der Kultur zugegeben wird; und das Screenen auf eine Infektion umfasst, wobei die Gegenwart oder Abwesenheit infizierter Zellen auf die von dem HIV verwendeten Korezeptoren hinweist.

2. Verfahren nach Anspruch 1, wobei das Viruspräparat in Gegenwart eines Antikoagulans entnommen wird, das mit dem zugegebenen Antikoagulans identisch oder von diesem verschieden sein kann.

3. Verfahren nach Anspruch 1 oder 2, wobei das Antikoagulans aus einem Antikoagulans, einem Thrombozytenaggregationshemmer, einem Thrombolytikum oder Fibrinolytikum und einem Mittel, bei dem es sich nicht um ein Arzneimittel handelt, ausgewählt ist.

4. Verfahren nach Anspruch 3, wobei das Antikoagulans Heparin, Citratdextrose (ACD), EDTA, eine Kombination von Heparin und ACD oder EDTA oder eine Kombination von Heparin, ACD und EDTA ist.

5. Verfahren nach einem der Ansprüche 1 bis 4, wobei das Plasma aus Vollblut stammt, das in Gegenwart eines Antikoagulans entnommen wurde.

6. Verfahren nach einem der Ansprüche 1 bis 5, wobei der Kultivierungsschritt ferner ein kationisches Polymer umfasst.

7. Verfahren nach Anspruch 6, wobei das kationische Polymer Polybren ist.

8. Verfahren nach einem der Ansprüche 1 bis 7, wobei das Verfahren das Entnehmen von Blut von dem Subjekt in Gegenwart eines Antikoagulans umfasst, um Plasma aus dem Blut zur Verwendung in dem Verfahren zu erzeugen.

9. Verfahren zum Auswählen eines Virusantagonisten, der zur Behandlung eines Subjekts, das mit HIV infiziert ist, geeignet ist, wobei das Verfahren das Bestimmen der Korezeptor-Verwendung durch das Verfahren nach einem der Ansprüche 1 bis 8 und das Auswählen eines Virusantagonisten auf Basis der Korezeptor-Verwendung umfasst.

10. Verfahren zum Bestimmen der Viruslast in einem Subjekt, wobei das Verfahren das Kultivieren eines Virus, das aus Plasma von dem Subjekt isoliert wurde, mit Zellen, die von dem Virus infiziert werden können, das Zugeben eines Antikoagulans zu der Virus/Zell-Kultur, das Screenen auf eine Infektion und dann das Bestimmen der Virusgenom-Kopiezahl in infizierten Zellen oder Kulturüberstand umfasst.

11. Verfahren nach Anspruch 10, wobei das Virus HIV ist.

12. Verfahren nach einem der Ansprüche 1 bis 11, wobei das HIV HIV-1 ist.

13. Verfahren nach einem der Ansprüche 10 bis 12, wobei das Antikoagulans aus einem Antikoagulans, einem Thrombozytenaggregationshemmer, einem Thrombolytikum oder Fibrinolytikum und einem Mittel, bei dem es sich nicht um ein Arzneimittel handelt, ausgewählt ist.

14. Verfahren nach einem der Ansprüche 5 bis 13, wobei das Antikoagulans Heparin, ACD und/oder EDTA ist.

15. Verfahren nach Anspruch 1, 8, 9 oder 13, wobei das Antikoagulans kein Metallionenchelator ist.

16. Verfahren nach Anspruch 1, 8, 9 oder 13, wobei das Antikoagulans nicht ein Metallionenchelator ist, der aus EDTA, ACD und Oxalat ausgewählt ist.

17. Verfahren nach einem der Ansprüche 10 bis 16, wobei die Virusgenom-Kopiezahl mittels RT-PCR bestimmt wird.

18. Verwendung von einem oder mehreren Antikoagulantien und Indikatorzelllinien, die Oberflächen-CD4 und einen oder beide von CCR4 und CXCR5 exprimieren, bei der Herstellung eines Assays zum Bestimmen der Korezeptor-Verwendung von HIV oder zum Bestimmen der Viruslast.

19. Assaykit zum Bestimmen der Korezeptor-Verwendung von HIV oder zum Bestimmen der Viruslast, wobei das Kit ein oder mehrere Antikoagulantien und Indikatorzelllinien, die Oberflächen-CD4 und einen oder mehrere von CCR4 und CXCR5 exprimieren, umfasst.

20. Verfahren, Verwendung oder Assaykit nach einem der Ansprüche 1 oder 8 bis 19, wobei die Zelllinien aus GHOSTX4R5- und TZMb1-Zellen ausgewählt sind.

21. Verfahren nach einem der Ansprüche 1 bis 17 unter Verwendung eines Assaykits nach Anspruch 19.

## Revendications

1. Procédé de détermination d'un usage comme co-récepteur d'un VIH chez un sujet, ledit procédé comprenant la culture d'une préparation virale tirée du plasma dudit sujet avec une ou plusieurs lignées cellulaires choisies parmi :
(i) des cellules qui expriment un CD4 et un CCR5 de surface ;
(ii) des cellules qui expriment un CD4 et un CXCR4 de surface ; et
(iii) des cellules qui expriment un CD4, un CCR5 et un CXCR4 de surface, dans lequel un anticoagulant est ajouté à la préparation virale ou à la culture ; et
le dépistage d'une infection, dans lequel la présence ou l'absence de cellules infectées est indicative des co-récepteurs utilisés par le VIH.

2. Procédé selon la revendication 1, dans lequel la préparation virale est recueillie en présence d'un agent anticoagulant qui peut être le même que ou différent de l'anticoagulant ajouté.

3. Procédé selon la revendication 1 ou la revendication 2, dans lequel l'anticoagulant est choisi parmi un anticoagulant, un médicament anti-plaquettes, un médicament thrombolytique ou fibrinolytique et un agent non médicinal.

4. Procédé selon la revendication 3, dans lequel l'anticoagulant est l'héparine, le dextrose de citrate (ACD), l'EDTA, une combinaison d'héparine et d'ACD ou d'EDTA, ou une combinaison d'héparine, d'ACD et d'EDTA.

5. Procédé selon l'une quelconque des revendications 1 à 4, dans lequel le plasma est tiré de sang entier recueilli en présence d'un anticoagulant.

6. Procédé selon l'une quelconque des revendications 1 à 5, dans lequel l'étape de culture comprend par ailleurs un polymère cationique.

7. Procédé selon la revendication 6, dans lequel le polymère cationique est le polybrène.

8. Procédé selon l'une quelconque des revendications 1 à 7, dans lequel ledit procédé comprend la collecte de sang auprès dudit sujet en présence d'un anticoagulant pour générer du plasma provenant du sang pour un usage dans le procédé.

9. Procédé de sélection d'un antagoniste viral convenant au traitement d'un sujet infecté du VIH, ledit procédé comprenant la détermination de l'usage comme co-récepteur par le procédé de l'une quelconque des revendications 1 à 8 et la sélection d'un antagoniste viral sur la base d'un usage de co-récepteur.

10. Procédé de détermination de charge virale chez un sujet, le procédé comprenant la culture d'un virus isolé du plasma du sujet avec des cellules capables d'être infectées par le virus, l'addition d'un anticoagulant à la culture de virus/cellules, le dépistage de l'infection et ensuite la détermination du nombre de copies du génome viral dans les cellules infectées ou dans le surnageant de culture.

11. Procédé selon la revendication 10, dans lequel le virus est le VIH.

12. Procédé selon l'une quelconque des revendications 1 à 11, dans lequel le VIH est le VIH-1.

13. Procédé selon l'une quelconque des revendications 10 à 12, dans lequel l'anticoagulant est choisi parmi un anticoagulant, un médicament anti-plaquettes, un médicament thrombolytique ou fibrinolytique ou un agent non médicinal.

14. Procédé selon l'une quelconque des revendications 5 à 13, dans lequel l'anticoagulant est l'héparine, l'ACD et/ou l'EDTA.

15. Procédé selon la revendication 1, 8, 9 ou 13, dans lequel l'anticoagulant n'est pas un chélateur d'ion métallique.

16. Procédé selon la revendication 1, 8, 9 ou 13, dans lequel l'anticoagulant n'est pas un chélateur d'ion métallique choisi parmi l'EDTA, l'ACD et l'oxalate.

17. Procédé selon l'une quelconque des revendications 10 à 16, dans lequel le nombre de copies du génome viral est déterminé par RT-PCR.

18. Utilisation d'un ou plusieurs anticoagulants et de lignées cellulaires indicatrices exprimant le CD4 de surface et un ou les deux CCR4 et CXCR5 dans la fabrication d'un essai pour déterminer l'usage comme co-récepteur du VIH et pour déterminer la charge virale.

19. Trousse d'essai pour déterminer l'usage comme co-récepteur du VIH ou pour déterminer la charge virale, dans laquelle la trousse comprend un ou plusieurs anticoagulants et des lignées cellulaires indicatrices exprimant le CD4 de surface et un ou plusieurs de CCR4 et CXCR5.

20. Procédé, utilisation ou trousse d'essai selon l'une quelconque des revendications 1 ou 8 à 19, dans lequel ou laquelle les lignées cellulaires sont choisies parmi les cellules GHOSTX4R5 et TZMb1.

21. Procédé selon l'une quelconque des revendications 1 à 17 utilisant une trousse d'essai telle que revendiquée dans la revendication 19.
